# EUROPEAN PATENT APPLICATION

(11) **EP 4 372 521 A1**
(43) Date of publication of application: **22.05.2024**
(21) Application number: 22856219.5
(22) Date of filing: 10.08.2022
(51) Int. Cl.: G06F 1/16, G06F 3/041, G06V 40/20, G10L 25/03

(54) **WEARABLE ELECTRONIC DEVICE AND METHOD BY WHICH WEARABLE ELECTRONIC DEVICE PROVIDES BRUSHING TEETH INFORMATION**

(30) Priority: 11.08.2021 KR 20210105842
(71) Applicant: Samsung Electronics Co., Ltd., Suwon-si, Gyeonggi-do 16677 (KR)
(72) Inventor: KIM, Hyeonseong, Suwon-si Gyeonggi-do 16677 (KR); PARK, Jeongmin, Suwon-si Gyeonggi-do 16677 (KR)
(74) Representative: HGF
(86) International application number: PCT/KR2022/011922
(87) International publication number: WO 2023/018222

(57) **Abstract**

According to an embodiment, a wearable electronic device comprises a motion sensor, an audio sensor, a display, a memory, and a processor electrically connected to the motion sensor, the audio sensor, and the memory, wherein the processor may be configured to: obtain motion sensing information through the motion sensor; obtain an audio signal corresponding to the motion sensing information through the audio sensor; identify a brushing teeth hand motion type corresponding to the motion sensing information; identify an audio signal pattern corresponding to the brushing teeth motion type; and identify a brushing teeth hand motion corresponding to the motion sensing information and the audio signal on the basis of the brushing teeth hand motion type and the audio signal pattern, and other embodiments may be possible.

## Description

### [Technical Field]

One or more embodiments relate to a wearable electronic device and a method for providing tooth-brushing information in the wearable electronic device.

### [Background Art]

Recently, electronic devices may be equipped with various functions. For example, not only a communication function, but also an entertainment function (for example, gaming), a multimedia function (for example, music/video playback), communication and security functions for mobile banking and the like, a scheduling function, and an electronic wallet function may be integrated into a single electronic device. Such electronic devices have become compact such that users can conveniently carry and wear the same. In line with development of electronic/communication technologies, such electronic devices have become compact/lightweight such that the same can be used without considerable inconvenience even when worn on human bodies.

Wearable electronic devices have been used increasingly as electronic devices which are convenient to use in daily life, and which are of portable or wearable types. For example, wearable electronic devices may be implemented in various types, including accessories such as eyeglasses, watches, and rings, clothes, or body implants, and may collect and provide detailed information regarding peripheral environments or physical changes of individuals in real time.

### [Detailed Description of the Invention]

### [Technical Problem]

Users may want information related to tooth brushing as detailed information regarding peripheral environments or physical information of individuals. Conventional devices such as smart toothbrushes may be used to provide information related to the users' tooth brushing. However, smart toothbrushes may need to be replaced periodically, and users may use different toothbrushes depending on the place and situation, thereby making it inconvenient to use the same to obtain information related to tooth brushing regardless of the time and place. In addition, information related to tooth brushing that may be provided conventionally may have a low level of reliability because of the low level of accuracy in recognition of the tooth-brushing situation.

Therefore, it would be convenient for users to be able to obtain information related to tooth brushing through a wearable electronic device which is convenient to carry in daily life, and which is of portable or wearable type. Furthermore, if the tooth-brushing situation can be recognized more accurately through an advanced sensing scheme, information related to tooth brushing may be provided at a higher level of reliability.

An embodiments may provide a wearable electronic device and a method wherein various and accurate information related to tooth brushing may be identified by sensing motions and sounds in daily life.

An embodiments may provide a wearable electronic device and a method wherein, when a user brushes teeth, the type of the tooth-brushing motion (for example, vertical tooth brushing or horizontal tooth brushing) may be accurately recognized, and various and specific tooth brushing-related guide information may be provided based on the type of the tooth-brushing motion.

### [Technical Solution]

A wearable electronic device according to an embodiment may include a motion sensor, an audio sensor, a display, a memory, and a processor electrically connected to the motion sensor, the audio sensor, and the memory, wherein the processor is configured to obtain motion sensing information via the motion sensor, obtain an audio signal corresponding to the motion sensing information via the audio sensor, identify a tooth-brushing hand motion type corresponding to the motion sensing information, identify an audio signal pattern corresponding to the tooth-brushing hand motion type, and identify, based on the tooth-brushing hand motion type and the audio signal pattern, a tooth-brushing hand motion corresponding to the motion sensing information and the audio signal.

A method for providing tooth-brushing information in a wearable electronic device according to an embodiment may include obtaining motion sensing information via a motion sensor of the wearable electronic device, obtaining an audio signal corresponding to the motion sensing information via an audio sensor of the wearable electronic device, identifying a tooth-brushing hand motion type corresponding to the motion sensing information, identifying an audio signal pattern corresponding to the tooth-brushing hand motion type, and identifying, based on the tooth-brushing hand motion type and the audio signal pattern, a tooth-brushing hand motion corresponding to the motion sensing information and the audio signal.

In connection with a non-transitory storage medium storing instructions according to an embodiment, the instructions may be configured to enable, when executed by at least one processor of a wearable electronic device, the wearable electronic device to perform at least one operation, and the at least one operation may include obtaining motion sensing information via a motion sensor of the wearable electronic device, obtaining an audio signal corresponding to the motion sensing information via an audio sensor of the wearable electronic device, identifying a tooth-brushing hand motion type corresponding to the motion sensing information, identifying an audio signal pattern corresponding to the tooth-brushing hand motion type, and identifying a tooth-brushing hand motion corresponding to the motion sensing information and the audio signal, based on the tooth-brushing hand motion type and the audio signal pattern.

### [Advantageous Effects]

According to various embodiments, a wearable device may be used to identify various and more accurate information related to tooth brushing by sensing motions and sounds in daily life.

According to various embodiments, when a user brushes teeth, a wearable device may be used to recognize the type of the tooth-brushing motion (for example, vertical tooth brushing or horizontal tooth brushing or other type of tooth brushing) more accurately, and various and specific tooth brushing-related guide information may be provided based on the type of the tooth-brushing motion.

Advantageous effects obtainable from the disclosure are not limited to the above-mentioned advantageous effects, and other advantageous effects not mentioned herein will be clearly understood by those skilled in the art to which the disclosure pertains.

### [Brief Description of Drawings]

FIG. 1 is a block diagram of an electronic device in a network environment according to an embodiment.
FIG. 2 is a block diagram of a wearable electronic device according to an embodiment.
FIG. 3A is a view illustrating an example of a first wearable electronic device according to an embodiment.
FIG. 3B is a view illustrating an example of a second wearable electronic device according to an embodiment.
FIG. 4 is a view illustrating an example of an internal configuration of a second wearable electronic device according to an embodiment.
FIG. 5 is a flowchart showing an operation of providing tooth-brushing information in a wearable electronic device according to an embodiment.
FIG. 6 is a view illustrating an example of a tooth-brushing session from the start of brushing to the end of brushing according to an embodiment.
FIG. 7 is a flowchart showing an operation of identifying a tooth-brushing hand motion type in a wearable electronic device according to an embodiment.
FIG. 8A is a view illustrating an example of a first acceleration sensor data graph when brushing teeth in a horizontal direction according to an embodiment.
FIG. 8B is a view illustrating an FFT transform graph with respect to a first acceleration sensor data when brushing teeth in a horizontal direction according to an embodiment.
FIG. 9A is a view illustrating an example of a second acceleration sensor data graph when brushing teeth in a vertical direction according to an embodiment.
FIG. 9B is a view illustrating an FFT transform graph with respect to a second acceleration sensor data when brushing teeth in a vertical direction according to an embodiment.
FIG. 10A is a view illustrating an example of a third acceleration sensor data graph when drying hair according to an embodiment.
FIG. 10B is a view illustrating an FFT transform graph with respect to a third acceleration sensor data when drying hair according to an embodiment.
FIG. 11A is a view illustrating an example of a fourth acceleration sensor data graph when washing hands according to an embodiment.
FIG. 11B is a view illustrating an FFT transform graph with respect to a fourth acceleration sensor data when washing hands according to an embodiment.
FIG. 12 is a view for illustrating a machine learning-based classifier model according to an embodiment.
FIG. 13 is a flowchart showing a tooth-brushing sound identification operation using an audio signal pattern type corresponding to a tooth-brushing hand motion type in a wearable electronic device according to an embodiment.
FIG. 14A is a view illustrating an example of an audio signal graph obtained when brushing teeth in a horizontal direction according to an embodiment.
FIG. 14B is a view illustrating an example of an audio signal graph obtained when brushing teeth in a vertical direction according to an embodiment.
FIG. 14C is a view illustrating an example of an audio signal graph obtained when washing hands according to an embodiment.
FIG. 15A is a graph showing the result of learning, by using a GMM model, two best frequency components among various local maxima frequency features in a single-sided amplitude spectrum in an audio signal during tooth-brushing in which the motion in a horizontal direction and the motion in a vertical direction are mixed according to an embodiment.
FIG. 15B is a graph showing the result of learning, by using a GMM model, two best frequency components among various local maxima frequency features in a single-sided amplitude spectrum in an audio signal when washing hands according to an embodiment.
FIG. 16A is a graph showing the result of learning, by using a GMM model, two best frequency components among various local maxima frequency features in a single-sided amplitude spectrum in an audio signal when brushing teeth in a horizontal direction according to an embodiment.
FIG. 16B is a graph showing the result of learning, by using a GMM model, two best frequency components among various local maxima frequency features in a single-sided amplitude spectrum in an audio signal when brushing teeth in a vertical direction according to an embodiment.
FIG. 17A is a view illustrating an example of a first information display related to tooth-brushing in a wearable electronic device according to an embodiment.
FIG. 17B is a view illustrating an example of a second information display related to tooth-brushing in a wearable electronic device according to an embodiment.
FIG. 17C is a view illustrating an example of a third information display related to tooth-brushing in a wearable electronic device according to an embodiment.
FIG. 17D is a view illustrating an example of a fourth information display related to tooth-brushing in a wearable electronic device according to an embodiment.
FIG. 17E is a view illustrating an example of a fifth information display related to tooth-brushing in a wearable electronic device according to an embodiment.
FIG. 17F is a view illustrating an example of a sixth information display related to tooth-brushing in a wearable electronic device according to an embodiment.

In connection with the description of the drawings, the same or similar reference numerals may be used for the same or similar components.

### [Mode for Carrying out the Invention]

In the following description of embodiments of the present disclosure, detailed descriptions of known functions or configurations are omitted in order to avoid unnecessarily obscuring the subject matter of the present disclosure. Throughout the drawings, like reference numerals will be understood to refer to like parts, components, and structures. The terms used herein are only used to describe specific embodiments and may not be intended to limit the present disclosure. An expression in a singular form may include an expression in a plural form unless the context clearly indicates otherwise. All terms used herein, including technical or scientific terms, may have the same meaning as commonly understood by a person ordinarily skilled in the art to which the present disclosure belongs. Terms defined in a commonly used dictionary may be interpreted as having the same or similar meaning as the meaning in the context of the related art, and unless explicitly defined in this document, are not interpreted in an ideal or excessively formal meaning. In some cases, even terms defined in this document cannot be construed to exclude embodiments of the disclosure.

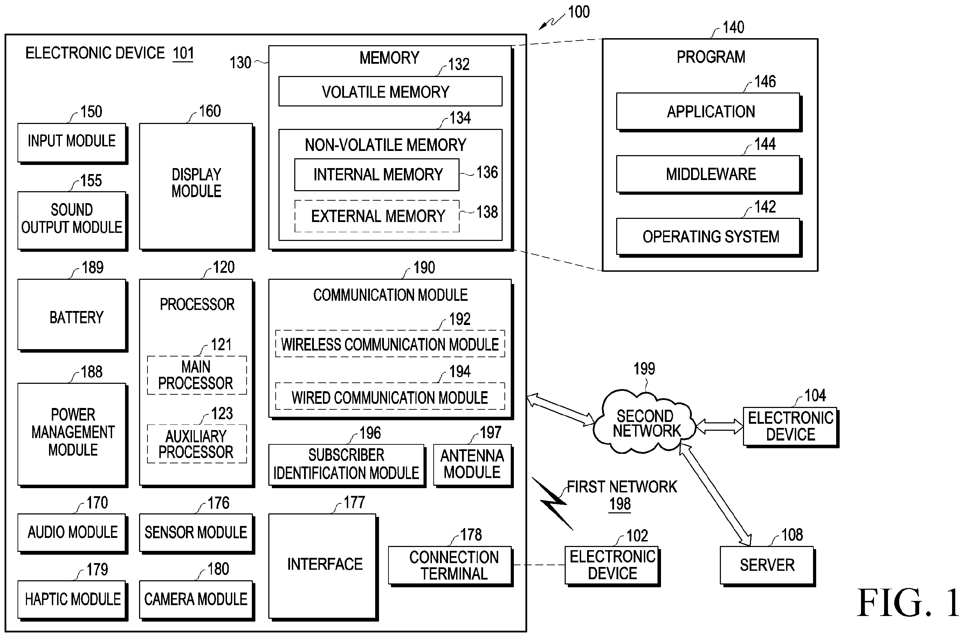

FIG. 1 is a block diagram illustrating an electronic device 101 in a network environment 100 according to an embodiment.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control, for example, at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active (e.g., executing an application) state. According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence model is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or an external electronic device (e.g., an electronic device 102 (e.g., a speaker or a headphone)) directly or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing 1eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example.

The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

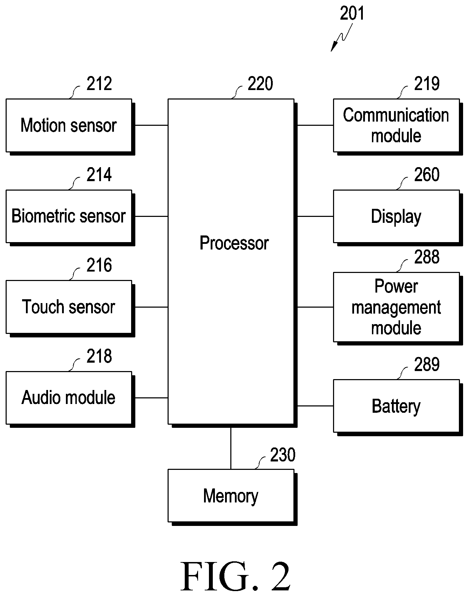

FIG. 2 is a block diagram of a wearable electronic device according to an embodiment.

Referring to FIG. 2, a wearable electronic device 201 (e.g., an electronic device 101 in FIG. 1) according to an embodiment may include some or all of a motion sensor 212, a biometric sensor 216, an audio module 218, a communication module 219, a processor 220, a memory 230, a display 260, a power management module 288, and a battery 289.

The motion sensor (or an inertia sensor) 212 according to an embodiment may sense motion of the electronic device 201 or motion of a user body (e.g., arm, finger, or wrist) wearing (or carrying) the electronic device 201. For example, the motion sensor 212 may include an acceleration sensor and/or a gyro sensor and may further include a geomagnetic sensor. The acceleration sensor may sense the impact or acceleration experienced by the electronic device 201 or caused by the movement of the body of the user wearing the electronic device 201. The gyro sensor may sense a rotation angle or a rotation direction of the electronic device 201, which is experienced by the electronic device 201 or caused by the movement of the body of the user wearing the electronic device 201. The geomagnetic sensor may sense direction of geomagnetism. For example, the processor 220 may use the acceleration sensor to identify whether the electronic device 201 or the body of the user wearing the electronic device 201 moves, and may use the gyro sensor to identify the rotation angle or the rotation direction caused by the movement of the body. For example, the proceeding direction of the motion of the body may be identified by using the geomagnetic sensor.

The biometric sensor 214 according to an embodiment may include at least one biometric sensor. The biometric sensor may be a sensor which can sense a biometric signal of the user. The biometric sensor 214 according to an embodiment may provide the processor 220 with a biometric signal measured by sensing the biometric signal of the user or information (value or numerical value) (e.g., blood oxygen saturation value, body temperature value, electrocardiogram, excited state value, and degree of hydration and/or dehydration) based on the biometric signal measured by sensing the biometric signal of the user. For example, the biometric sensor 214 may include a photoplethysmography (PPG) sensor, and in addition, may further include a body temperature sensor, an electrocardiogram (ECG) sensor, an electrodermal activity (EDA) sensor, and/or a SWEAT sensor. In addition, it may further include other sensors associated with biosensing. The biometric sensor 214 according to an embodiment may provide the processor 220 with a biometric signal measured by sensing the biometric signal of the user or information (e.g., value or numerical value) (e.g., blood oxygen saturation value, body temperature value, electrocardiogram, excited state value, and degree of hydration and/or dehydration) based on the biometric signal measured by sensing the biometric signal of the user.

The touch sensor 216 according to an embodiment may detect touches. For example, the touch sensor 216 may detect a touch caused by the contact of the body of the user. The touch sensor 216 according to an embodiment may be included in the display 260 or may be included in the electronic device 201 as a separate element.

The audio module 218 according to an embodiment may convert sound into an electrical signal, or vice versa. According to an embodiment, the audio module 218 may acquire sound through a microphone (not illustrated) or may convert digital information (e.g. an audio file) into sound to output the sound through a speaker (not illustrated).

The communication module 219 according to an embodiment may communicate with an external electronic device (e.g., an external electronic device 102 in FIG. 1). According to an embodiment, the communication module 219 may transfer information (or data) obtained by the electronic device 201 to an external electronic device 102 or receive information (or data) from the external electronic device 102 based on the control by the processor 220. For example, the communication module 219 may include a cellular communication module, an ultra-wide band (UWB) communication module, a Bluetooth communication module, and/or a wireless fidelity (WiFi) communication module, and in addition, may further include other modules which can communicate with the external electronic device 102.

The processor 220 according to an embodiment may be electrically connected to the motion sensor 212, the biometric sensor 214, the touch sensor 216, the audio module (or an audio sensor) 218, the communication module 219, the memory 230, the display 260, and/or the power management module 288.

The processor 220 according to an embodiment may obtain motion sensing information from the motion sensor 212. The motion sensor 212 may be turned on by a user input, may be turned on by a designated configuration information, or always be on. The motion sensing information may include acceleration information sensed by the acceleration sensor, rotation direction or rotation angle information sensed by the gyro sensor, and geomagnetic sensing information sensed by the geomagnetic sensor. For example, the acceleration information may include the acceleration value sensed by the acceleration sensor when the electronic device 201 or the body of the user wearing the electronic device 201 moves. The rotation direction or rotation angle information may include the rotation direction or rotation angle valve of the electronic device 201, which is sensed by the gyro sensor when the electronic device 201 or the body of the user wearing the electronic device 201 moves. The geomagnetic sensing information may include direction of geomagnetism to indicate the direction of the movement.

The processor 220 according to an embodiment may identify the start of tooth-brushing (or detect the tooth-brushing hand motion) based on the obtained motion sensing information. The processor 220 according to an embodiment may identify whether the obtained motion sensing information corresponds to a first tooth-brushing hand motion pattern and/or corresponds to a second tooth-brushing hand motion pattern by using at least one pre-stored tooth-brushing hand motion information (or at least one tooth-brushing hand motion pattern) (e.g., first tooth-brushing hand motion information (or pattern) and/or second tooth-brushing hand motion information (or pattern)). For example, the processor 220 may identify whether the obtained motion sensing information corresponds to the first tooth-brushing hand motion pattern and/or corresponds to the second tooth-brushing hand motion pattern by using the tooth-brushing hand motion type information previously obtained by using a machine learning-based classifier model.

The processor 220 according to an embodiment may identify (or detect the tooth-brushing hand motion) that tooth-brushing has started when the obtained motion sensing information corresponds to the first tooth-brushing hand motion pattern and/or the second tooth-brushing hand motion pattern. For example, the first tooth-brushing hand motion pattern may include a tooth-brushing pattern (e.g. a pattern of brushing teeth in left and right directions) in a first direction (a horizontal direction). The second tooth-brushing hand motion pattern may include a tooth-brushing pattern (e.g. a pattern of brushing teeth in upward and downward directions) in a second direction (a vertical direction). According to an embodiment, the first tooth-brushing hand motion pattern and/or the second tooth-brushing hand motion pattern may be obtained by using a pre-trained machine-learning based classifier model.

The processor 220 according to an embodiment may turn on the audio module 218 (e.g., turn on an audio sensor) in response to identifying (or detecting the tooth-brushing hand motion) the start of tooth-brushing so as to obtain audio signal from the audio module 218. The processor 220 according to an embodiment may obtain an audio signal from the audio module 218 while obtaining the motion sensing information from the motion sensor 212 after identifying the start of brushing.

The processor 220 according to an embodiment may identify the tooth-brushing hand motion corresponding to a first audio signal and the first motion sensing information, based on the obtained first motion sensing information and the first audio signal corresponding (or obtained together with the first motion sensing information) to the first motion sensing information, after identifying (or detecting the tooth-brushing hand motion) the start of brushing. For example, the type of tooth-brushing hand motion may include a first tooth-brushing hand motion and/or a second tooth-brushing hand motion. The first tooth-brushing hand motion may be a motion corresponding to the tooth-brushing (e.g. brushing teeth in left and right directions) in a first direction (horizontal direction) (e.g. brushing teeth in left and right directions). The second tooth-brushing hand motion may include the tooth-brushing (e.g. brushing teeth in upward and downward directions) in a second direction (vertical direction) (e.g. brushing teeth in upward and downward directions). For example, the type of tooth-brushing hand motion may further include brushing teeth in a circle (or brushing teeth while drawing a circle on the teeth) as a third tooth-brushing hand motion or may further include other types of tooth-brushing hand motions in addition to the above examples.

The processor 220 according to an embodiment may identify whether the first motion sensing information corresponds to the first tooth-brushing hand motion pattern and whether the first audio signal corresponding to the first motion sensing information corresponds to first tooth-brushing sound pattern (e.g., a first audio signal pattern) related to the first tooth-brushing hand motion pattern. The processor according to an embodiment may determine (confirm or identify) that the first motion sensing information and the first audio signal correspond to the first tooth-brushing hand motion pattern when the first motion sensing information corresponds to the first tooth-brushing hand motion pattern and the first audio signal corresponds to the first tooth-brushing sound pattern (e.g., the first audio signal pattern) related to the first tooth-brushing hand motion pattern. The processor 220 according to an embodiment may ignore the first motion sensing information or may determine (confirm or identify) that the first motion sensing information and the first audio signal do not correspond to the first tooth-brushing hand motion when the first motion sensing information corresponds to the first tooth-brushing hand motion pattern but the first audio signal does not correspond to the first tooth-brushing sound pattern (e.g., the first audio signal pattern) related to the first tooth-brushing hand motion pattern.

The processor 220 according to an embodiment may identify whether the first audio signal corresponding to the first motion sensing information corresponds to a second tooth-brushing sound pattern (e.g., a second audio signal pattern) related to the second tooth-brushing hand motion pattern when the first motion sensing information corresponds to the second tooth-brushing hand motion pattern. The processor according to an embodiment may determine (confirm or identify) that the first motion sensing information and the first audio signal correspond to the second tooth-brushing hand motion when the first motion sensing information corresponds to the second tooth-brushing hand motion pattern and the first audio signal corresponds to the second tooth-brushing sound pattern (e.g., the second audio signal pattern) related to the second tooth-brushing hand motion pattern. The processor 220 according to an embodiment may ignore the first motion sensing information or may determine (confirm or identify) that the second tooth-brushing hand motion has not occurred when the first motion sensing information corresponds to the second tooth-brushing hand motion pattern, but the first audio signal does not correspond to the second tooth-brushing sound pattern (e.g., the second audio signal pattern) related to the second tooth-brushing hand motion pattern.

The processor 220 according to an embodiment may identify a tooth-brushing hand motion corresponding to second motion sensing information and a second audio signal in a manner similar to the processing method of the first motion sensing information and the first audio signal when the second motion sensing information and the second audio signal obtained after the first motion sensing information and the first audio signal are present. The processor may identify a tooth-brushing hand motion corresponding to third motion sensing information and a third audio signal when the third motion sensing information and the third audio signal obtained after the second motion sensing information and the second audio signal are present. The processor 220 according to an embodiment may determine (confirm or identify) that the tooth-brushing is over and end the tooth-brushing hand motion identification operation when the motion sensing information and the audio signal are not detected for a designated time period. According to another embodiment, even when the motion sensing information and the audio signal are continuously detected, the processor 220 may determine (confirm or identify) that the tooth-brushing is over and end the tooth-brushing hand motion identification operation when the motion sensing information and the audio signal are related to another behavior pattern (e.g., walking of a user) of a user.

The processor 220 according to an embodiment may obtain and store information related to tooth-brushing from the start of brushing to the end of brushing. For example, the processor 220 may store tooth-brushing time from the start of brushing to the end of brushing, a tooth-brushing hand motion pattern for the tooth-brushing time (e.g., the first tooth-brushing hand motion -> the second tooth-brushing hand motion -> the first tooth-brushing hand motion or the second tooth-brushing hand motion -> the first tooth-brushing hand motion -> the second tooth-brushing hand motion), the number of times of the first and/or second tooth-brushing hand motions for the tooth-brushing time, and/or a tooth-brushing score based on a predetermined recommended tooth-brushing standard. In addition, it may further obtain and store other information related to tooth-brushing.

The processor 220 according to an embodiment may display information related to tooth-brushing from the start of brushing to the end of brushing via the display 260, and may further provide a guide (or an alarm) related to tooth-brushing based on information related to tooth-brushing. For example, the guide (or the alarm) related to tooth-brushing may include a message indicating that the recommended tooth-brushing time has not been reached (or has been exceeded), a message indicating that the recommended number of times of a first tooth-brushing motion and/or the recommended number of times of a second tooth-brushing motion have not been reached (or have been exceeded), and/or a message indicating that the recommended tooth-brushing standard have not been met (or have been exceeded). The processor 220 according to an embodiment may provide tooth-brushing history information based on a plurality of tooth-brushing histories when a plurality of tooth-brushings have been performed. The processor 220 according to an embodiment may transmit information related to the tooth-brushing from the start of brushing to the end of brushing, the guide (or the alarm) related to the tooth-brushing, and/or the tooth-brushing history information to an external electronic device 102 via the communication module 219. For example, the external electronic device 102 may be another electronic device (e.g., a smartphone or other wearable electronic device) linked with the electronic device 201 of the user, another electronic device of a family member of the user, or a server device in a dental hospital linked to the user, and the external device 102 may be another type of device.

The memory 230 (e.g., a memory 130 in FIG. 1) according to an embodiment may store various control data or obtained information used by at least one component (e.g., the processor 220 or another component) of the electronic device 201. According to an embodiment, the memory 230 may store instructions for executing the operation of the processor 220 of the electronic device 201. According to various embodiments, the memory 230 may be implemented in various forms such as read-only memory (ROM), random access memory (RAM), or flash memory, and there may be no limitation on the implementation form.

The display 260 according to an embodiment may display data or a screen under the control of the processor 220. For example, the display 260 may display a screen which displays at least a part of information related to tooth-brushing from the start of brushing to the end of brushing, guide or alarm related to the tooth-brushing, and/or the tooth-brushing history information under the control of the processor 220.

The power management module 288 according to an embodiment may be connected to the battery 289, may charge the battery 289 by using the power received from the outside, and may control the power from the battery 289 to be used by each element of the electronic device 201.

A wearable electronic device (e.g., an electronic device 101 in FIG. 1 or the electronic device 201 in FIG. 2) according to an embodiment may include a motion sensor (e.g., a sensor module 176 in FIG. 1 or the motion sensor 212 in FIG. 2), an audio sensor (e.g., an input module 150 in FIG. 1 or the audio module 218 in FIG. 2), a display (e.g., a display module 160 in FIG. 1 or the display 260 in FIG. 2), a memory (e.g., a memory 130 in FIG. 1 or the memory 230 in FIG. 2), and a processor (e.g., a processor 120 in FIG. 1 or the processor 220 in FIG. 2) electrically connected to the motion sensor, the audio sensor, the memory, wherein the processor is configured to obtain motion sensing information via the motion sensor, obtain an audio signal corresponding to the motion sensing information via the audio sensor, identify a tooth-brushing hand motion type corresponding to the motion sensing information, identify an audio signal pattern corresponding to the tooth-brushing hand motion type, and identify, based on the tooth-brushing hand motion type and the audio signal pattern, the tooth-brushing hand motion corresponding to the motion sensing information and the audio signal.

According to an embodiment, the tooth-brushing hand motion type may include a first tooth-brushing hand motion type and a second tooth-brushing hand motion type, and the processor may be configured to identify the first audio signal pattern corresponding to the first tooth-brushing hand motion type when the tooth-brushing hand motion type is the first tooth-brushing hand motion type, and identify the second audio signal pattern corresponding to the second tooth-brushing hand motion type when the tooth-brushing hand motion type is the second tooth-brushing hand motion type.

According to an embodiment, the first tooth-brushing hand motion type may indicate tooth-brushing hand motion in a horizontal direction, and the second tooth-brushing hand motion type may indicate tooth-brushing hand motion in a vertical direction.

According to an embodiment, the motion sensor may include an acceleration sensor, a gyro sensor, and a geomagnetic sensor, and the motion sensing information may include at least one of hand posture information, frequency characteristic information with respect to the tooth-brushing hand motion, and information about the magnitude and direction of the tooth-brushing hand motion which are obtained based on an acceleration value sensed by the acceleration sensor, a rotation direction or rotation angle value of the electronic device sensed by the gyro sensor, and geomagnetic direction information sensed by the geomagnetic sensor.

According to an embodiment, the processor may be configured to identify the start of tooth-brushing when a designated periodic motion pattern is identified based on the motion sensing information.

According to an embodiment, the wearable electronic device may further include a touch sensor and the processor may be configured to correct, based on the touch sensing information obtained by the touch sensor, a sensing axis of the motion sensor when the start of tooth-brushing is identified.

According to an embodiment, the processor may be configured to identify the end of tooth-brushing when the motion sensing information and/or the audio signal are not obtained for a designated time period.

According to an embodiment, the processor may be configured to control the display to display, based on the identification of the end of tooth-brushing, the information related to tooth-brushing from the start of tooth-brushing to the end of tooth-brushing on the display.

According to an embodiment, the wearable electronic device may further include a communication module, and the processor may be configured to transmit the information related to tooth-brushing to an external electronic device via the communication module.

According to an embodiment, the processor may be configured to identify the tooth-brushing hand motion type by comparing the tooth-brushing hand motion type information previously obtained by learning using a machine learning-based classifier model with the feature information extracted from the motion sensing information.

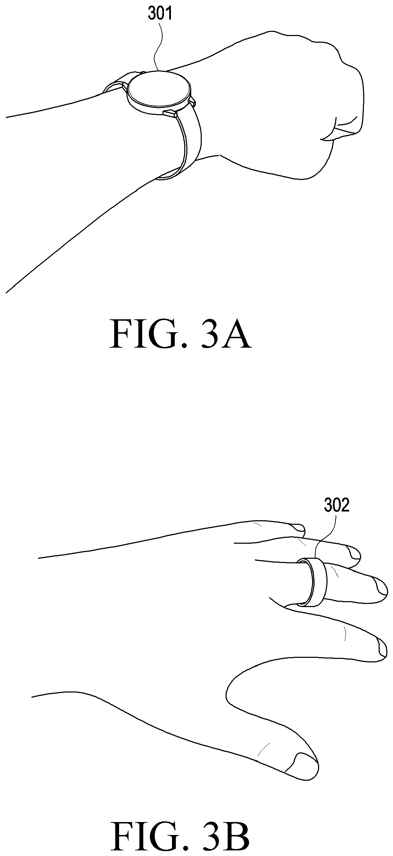

FIG. 3A is a view illustrating an example of a first wearable electronic device according to an embodiment, and FIG. 3B is a view illustrating an example of a second wearable electronic device according to an embodiment.

Referring to FIG. 3A and FIG. 3B, a wearable electronic device 201 according to an embodiment may include a first wearable electronic device 301 or a second wearable electronic device 302. For example, the first wearable electronic device 301 may be a smartwatch worn on a wrist of the user and the second wearable electronic device 302 may be a ring worn on a finger of the user. In addition, the wearable electronic device 201 may be in another form which can be worn, inserted or attached in a position (e.g., a body part other than the wrist or finger) where the user's hand motion can be easily sensed. For example, the wearable electronic device 201 may be a glove-type electronic device, a tattoo-type electronic device, or a body-insertion type electronic device. According to an embodiment, it is apparent to one of skill that the shape of the first wearable electronic device 301 or the second wearable electronic device 302 can be implemented in designs different from the design (or shape) illustrated in FIGS. 3A and 3B.

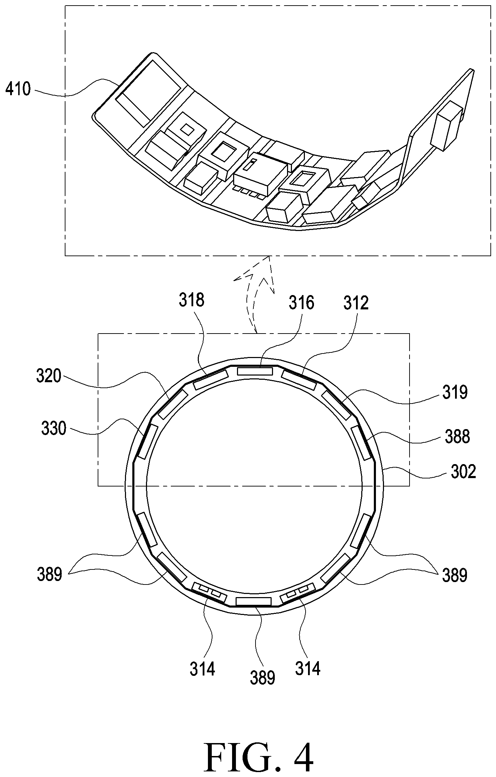

FIG. 4 is a view illustrating an example of an internal configuration of a second wearable electronic device according to an embodiment.

Referring to FIG. 4, the second wearable electronic device 302 according to an embodiment may include a motion sensor 312, a biometric sensor 314, a touch sensor 316, an audio module 318, a communication module 319, a processor 320, a memory 330, a power management module 388, and a battery 389. The wearable electronic device 302 according to an embodiment may include at least one flexible printed circuit board (FPCB) 410, and at least a part or all of the motion sensor 312, the biometric sensor 314, the touch sensor 316, the audio module 318, the communication module 319, the processor 320, the memory 330, the power management module 388, and the battery 389 may be arranged on at least one FPCB. According to an embodiment, the motion sensor 312, the biometric sensor 314, the touch sensor 316, the audio module 318, the communication module 319, the processor 320, the memory 330, the power management module 388, and the battery 389 may perform the same operations as the operations of the motion sensor 212, the biometric sensor 214, the touch sensor 216, the audio module 218, the communication module 219, the processor 220, the memory 230, the power management module 288, and the battery 289 of the electronic device 201 in FIG. 2. The positions at which at least a part of the motion sensor 312, the biometric sensor 314, the touch sensor 316, the audio module 318, the communication module 319, the processor 320, the memory 330, the power management module 388, and the battery 389 are disposed in the second wearable electronic device 302 can change depending on the embodiment.

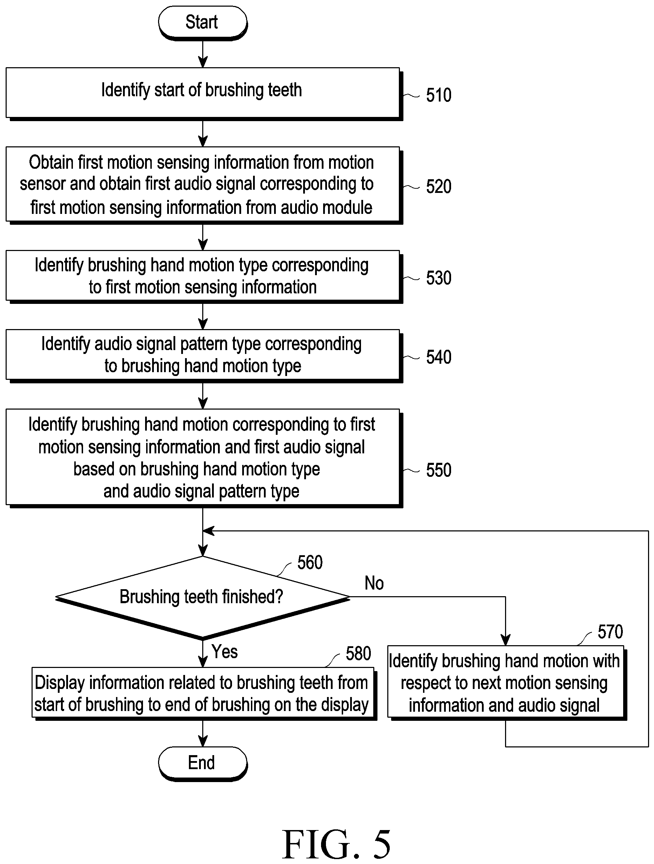

FIG. 5 is a flowchart showing an operation of providing tooth-brushing information in a wearable electronic device according to an embodiment.

Referring to FIG. 5, a processor (e.g., the processor 120 in FIG. 1, the processor 220 in FIG. 2, or the processor 320 in FIG. 4, and hereinafter, the processor 220 in FIG. 2 will be described as an example.) of an electronic device (e.g., the electronic device 101 in FIG. 1, the wearable electronic device 201 in FIG. 2, the first wearable electronic device 301 in FIG. 3A, and the second wearable electronic device 302 in FIG. 3B) according to an embodiment may perform at least one of operations 510 to 580.

In operation 510, the processor 220 according to an embodiment may identify (or detect the tooth-brushing hand motion) the start of brushing (or the start of a tooth-brushing session), based on the motion sensing information obtained from the motion sensor 212. The motion sensor 212 according to an embodiment may be activated by a user input, be activated by a designated configuration information, or always be in an activated state, and in the activated state, may perform the motion sensing operation to provide the processor 220 with the motion sensing information. For example, the motion sensor 212 may be woken up (e.g., activated from a low-power state to an activated state) by a driving signal (e.g., clock signal) by the processor 220, and may perform the motion sensing (monitoring) operation in the activated or wake-up state. According to an embodiment, the motion sensing information may include the acceleration information sensed by the acceleration sensor, the rotation direction or rotation angle information sensed by the gyro sensor, and geomagnetic sensing information sensed by the geomagnetic sensor. For example, the acceleration information may include the acceleration value sensed by the acceleration sensor when the electronic device 201 or the body of the user wearing the electronic device 201 moves. The rotation direction or rotation angle information may include the rotation direction or rotation angle valve of the electronic device 201, which is sensed by the gyro sensor when the electronic device 201 or the body of the user wearing the electronic device 201 moves. The geomagnetic sensing information may include direction of geomagnetism indicating the direction of the movement. For example, the processor 220 may obtain (or extract) information about the hand posture, the frequency characteristics with respect to the hand motion, and the magnitude and direction of the hand motion, during tooth-brushing, which are obtained via the acceleration value sensed by the acceleration sensor, the rotation direction or rotation angle value of the electronic device 201 sensed by the gyro sensor, and geomagnetic direction information sensed by the geomagnetic sensor. The processor 220 according to an embodiment may identify (or detect the tooth-brushing hand motion) the start of brushing (or the start of tooth-brushing session), based on the obtained information about the hand posture, the frequency characteristics with respect to a hand motion, and the size and direction of the hand motion during tooth-brushing.

For example, the processor 220 may identify whether the obtained motion sensing information corresponds to a first tooth-brushing hand motion pattern and/or corresponds to a second tooth-brushing hand motion pattern by using at least one pre-stored tooth-brushing hand motion information (or at least one tooth-brushing hand motion pattern) (e.g., the first tooth-brushing hand motion information (or pattern) and/or the second tooth-brushing hand motion information (or pattern)). The processor 220 according to an embodiment may identify (or detect the tooth-brushing hand motion) that the tooth-brushing has started when the obtained motion sensing information corresponds to the first tooth-brushing hand motion pattern and/or the second tooth-brushing hand motion pattern. For example, the first tooth-brushing hand motion pattern may include a tooth-brushing pattern (or a pattern of brushing teeth in left and right directions) in a first direction (horizontal direction) (e.g. pattern of brushing teeth in left and right directions). The second tooth-brushing hand motion pattern may include a tooth-brushing pattern (or a pattern of brushing teeth in upward and downward directions) in a second direction (vertical direction) (e.g. pattern of brushing teeth in upward and downward directions). According to an embodiment, the first tooth-brushing hand motion pattern and/or the second tooth-brushing hand motion pattern may be obtained by using a pre-trained machine-learning based classifier model.

In operation 520, in response to the identification of the start of brushing (or the detection of the tooth-brushing hand motion), the processor 220 according to an embodiment may obtain the first motion sensing information from the motion sensor 212 and obtain the first audio signal corresponding to the first motion sensing information from the audio module 218. According to an embodiment, the processor 220 may control the audio module 218 to be in an on-state (e.g., audio sensor is in an on-state) in response to the identification of the start of brushing (or the detection of the tooth-brushing hand motion).

In operation 530, the processor 220 may identify the tooth-brushing hand motion type corresponding to the first motion sensing information. For example, the tooth-brushing hand motion type may include a first tooth-brushing hand motion and/or a second tooth-brushing hand motion. The first tooth-brushing hand motion may be a motion corresponding to the tooth-brushing (e.g. brushing teeth in left and right directions) in a first direction (horizontal direction) (e.g. brushing teeth in left and right directions). The second tooth-brushing hand motion may include the tooth-brushing (e.g. brushing teeth in upward and downward directions) in a second direction (vertical direction) (e.g. brushing teeth in upward and downward directions). For example, the tooth-brushing hand motion type may further include brushing teeth in a circle (or brushing teeth while drawing a circle on the teeth) as a third tooth-brushing hand motion or may further include other types of tooth-brushing hand motions in addition to the above examples.

For example, the processor 220 may identify whether the first motion sensing information corresponds to the first tooth-brushing hand motion pattern or corresponds to the second tooth-brushing hand motion pattern by using at least one pre-stored tooth-brushing hand motion information (or at least one tooth-brushing hand motion pattern) (e.g., the first tooth-brushing hand motion information (or pattern) and/or the second tooth-brushing hand motion information (or pattern)). The processor 220 according to an embodiment may determine (confirm or identify) the tooth-brushing hand motion type corresponding to the first motion sensing information as the first tooth-brushing hand motion when the first motion sensing information corresponds to the first tooth-brushing hand motion pattern. The processor 220 according to an embodiment may determine (confirm or identify) the tooth-brushing hand motion type corresponding to the first motion sensing information as the second tooth-brushing hand motion when the first motion sensing information corresponds to the second tooth-brushing hand motion pattern.

In operation 540, the processor 220 according to an embodiment may identify an audio signal pattern type corresponding to the tooth-brushing hand motion type. For example, the audio signal pattern corresponding to the first tooth-brushing hand motion may be the first audio signal pattern and the audio signal pattern corresponding to the second tooth-brushing hand motion may be the second audio signal pattern. The processor 220 according to an embodiment may identify a first audio signal pattern type when the tooth-brushing hand motion type is the first tooth-brushing hand motion and may identify a second audio signal pattern type when the tooth-brushing hand motion type is the second tooth-brushing hand motion. For example, the first audio signal pattern type and/or the second audio signal pattern type may be the type acquired via an audio signal pattern analysis model by learning and modeling the audio signal pattern analysis model via a machine learning method.

In operation 550, the processor 220 according to an embodiment may identify, based on the tooth-brushing hand motion type and the audio signal pattern type, the tooth-brushing hand motion corresponding to the first motion sensing information and the first audio signal.

For example, the processor 220 may identify whether the first motion sensing information corresponds to the first tooth-brushing hand motion pattern and whether the first audio signal corresponding to the first motion sensing information corresponds to the first audio signal pattern related to the first tooth-brushing hand motion pattern. The processor according to an embodiment may determine (confirm or identify) that the first motion sensing information and the first audio signal correspond to the first tooth-brushing hand motion pattern when the first motion sensing information corresponds to the first tooth-brushing hand motion pattern and the first audio signal corresponds to the first audio signal pattern related to the first tooth-brushing hand motion pattern. The processor 220 may ignore the first motion sensing information or may determine (confirm or identify) that the first motion sensing information and the first audio signal do not correspond to the first tooth-brushing hand motion when the first motion sensing information corresponds to the first tooth-brushing hand motion pattern, but the first audio signal does not correspond to the first audio signal pattern.

The processor 220 according to an embodiment may identify whether the first audio signal corresponding to the first motion sensing information corresponds to the second audio signal pattern related to the second tooth-brushing hand motion pattern when the first motion sensing information corresponds to the second tooth-brushing hand motion pattern. The processor according to an embodiment may determine (confirm or identify) that the first motion sensing information and the first audio signal correspond to the second tooth-brushing hand motion when the first motion sensing information corresponds to the second tooth-brushing hand motion pattern and the first audio signal corresponds to the second audio signal pattern. The processor 220 according to an embodiment may ignore the first motion sensing information or may determine (confirm or identify) that the second tooth-brushing hand motion has not occurred when the first motion sensing information corresponds to the second tooth-brushing hand motion pattern, but the first audio signal does not correspond to the second audio signal pattern.

In operation 560, the processor 220 according to an embodiment may determine (confirm or identify) the end of tooth-brushing. The processor 220 according to an embodiment may determine (confirm or identify) that the tooth-brushing is ended when the motion sensing information and the audio signal are not obtained within a designated time period after the first motion sensing information and the first audio signal or when the audio signal related to the tooth-brushing is not obtained and the movement status such as walking of the user is recognized. The processor 220 according to an embodiment may determine (confirm or identify) that the tooth-brushing is not ended when the next motion sensing information and audio signal are obtained within a designated time period after the first motion sensing information and the first audio signal.

In operation 570, when the tooth-brushing is not ended, the processor 220 according to an embodiment may perform the tooth-brushing hand motion identification operation on the next motion sensing information and audio signal and return to the operation 560. For example, the processor 220 may identify the tooth-brushing hand motion corresponding to the second motion sensing information and the second audio signal in a manner similar to the processing method for the first motion sensing information and the first audio signal (e.g., manner similar to operations 520 to 550) when the second motion sensing information and the second audio signal are present after (or next to) the first motion sensing information and the first audio signal. The processor 220 may identify the tooth-brushing hand motion corresponding to the third motion sensing information and the third audio signal when the third motion sensing information and the third audio signal are present after (or next to) the second motion sensing information and the second audio signal. The processor 220 according to an embodiment may continue to identify the tooth-brushing hand motion from motion sensing information and audio signals related to tooth brushing until the motion sensing information and audio signals are not detected for a designated time period.

In operation 580, the processor 220 according to an embodiment may display information related to tooth-brushing from the start of brushing to the end of brushing via the display 260 when the tooth-brushing is ended. For example, the information related to tooth-brushing from the start of brushing to the end of brushing may include the time when tooth brushing started to the time when it ended, a tooth-brushing hand motion history for the tooth-brushing time (e.g., the first tooth-brushing hand motion -> the second tooth-brushing hand motion -> the first tooth-brushing hand motion or the second tooth-brushing hand motion -> the first tooth-brushing hand motion -> the second tooth-brushing hand motion), the number of times of the first and/or second tooth-brushing hand motions are performed during the tooth-brushing, and/or a tooth-brushing score based on a predetermined recommended tooth-brushing standard, and other information related to tooth-brushing may be further displayed. The processor 220 according to an embodiment may display the information related to the operations 510 to 570 on the display 260 until the end of brushing in the operation 580 or may display at least a part of the information related to the tooth-brushing from the start of brushing to the end of brushing. The processor 220 according to an embodiment may further display a guide or an alarm related to tooth-brushing based on the information related to the tooth-brushing. For example, the guide or alarm related to tooth-brushing may include a message indicating that the recommended tooth-brushing time has not been reached or has been exceeded, a message indicating that the recommended number of times of the first tooth-brushing motion and/or the second tooth-brushing motion are to be performed has not been reached or has been exceeded, and/or a message indicating that the recommended tooth-brushing standard have not been met or have been exceeded. The processor 220 according to an embodiment may display the tooth-brushing history information based on a plurality of tooth-brushing histories when a plurality of tooth-brushings have been performed. The processor 220 according to an embodiment may transmit information related to the tooth-brushing from the start of brushing to the end of brushing, the guide or the alarm related to the tooth-brushing, and/or the tooth-brushing history information to an external electronic device 102 via the communication module 219. For example, the external electronic device 102 may be another electronic device (e.g., a smartphone or other wearable electronic device) linked with the electronic device 201 of the user, other electronic device of a family member of the user, or a server device in a dental hospital linked to the user, and the external device 102 may be another type of device.

According to an embodiment, in a wearable electronic device (e.g., the electronic device 101 in FIG. 1, the wearable electronic device 201 in FIG. 2, the first wearable electronic device 301 in FIG. 3A, and the second wearable electronic device 302 in FIG. 3B), the method for providing the tooth-tooth-brushing information may include the steps of obtaining the motion sensing information via a motion sensor (e.g., the sensor module 176 in FIG. 1 or the motion sensor in FIG. 2) of the wearable electronic device, obtaining the audio signal corresponding to the motion sensing information via an audio sensor (e.g., the input module 150 in FIG. 1 or the audio module 218 in FIG. 2) of the wearable electronic device, identifying a tooth-brushing hand motion type corresponding to the motion sensing information, identifying an audio signal pattern corresponding to the tooth-brushing hand motion type, and identifying, based on the tooth-brushing hand motion type and the audio signal pattern, the tooth-brushing hand motion corresponding to the motion sensing information and the audio signal.

According to an embodiment, the tooth-brushing hand motion type may include a first tooth-brushing hand motion type and a second tooth-brushing hand motion type, and the method may further include the steps of identifying the first audio signal pattern corresponding to the first tooth-brushing hand motion type when the tooth-brushing hand motion type is the first tooth-brushing hand motion type, and identifying the second audio signal pattern corresponding to the second tooth-brushing hand motion type when the tooth-brushing hand motion type is the second tooth-brushing hand motion type.

According to an embodiment, the first tooth-brushing hand motion type may indicate tooth-brushing hand motion in a horizontal direction, and the second tooth-brushing hand motion type may indicate tooth-brushing hand motion in a vertical direction.

According to an embodiment, the motion sensor may include an acceleration sensor, a gyro sensor, and a geomagnetic sensor, and the motion sensing information may include at least one of hand posture information, frequency characteristic information with respect to the tooth-brushing hand motion, and information about the magnitude and direction of the tooth-brushing hand motion which are obtained based on an acceleration value sensed by the acceleration sensor, a rotation direction or rotation angle value of the electronic device sensed by the gyro sensor, and geomagnetic direction information sensed by the geomagnetic sensor.

According to an embodiment, the method may further include the steps of identifying the start of tooth-brushing when a designated periodic motion pattern is identified based on the motion sensing information.

According to an embodiment, the method may further include the step of correcting, based on the touch sensing information obtained from a touch sensor of the wearable electronic device, a sensing axis of the motion sensor when the start of tooth-brushing is identified.

According to an embodiment, it may further include the step of identifying the end of tooth-brushing when the motion sensing information and/or the audio signal are not obtained for a designated time period.

According to an embodiment, the method may further include the step of displaying, based on the identification of the end of tooth-brushing, the information related to tooth-brushing from the start of tooth-brushing to the end of tooth-brushing on a display of the wearable electronic device.

According to an embodiment, the method may further include the step of transmitting the information related to the tooth-brushing to an external electronic device via a communication module of the wearable electronic device.

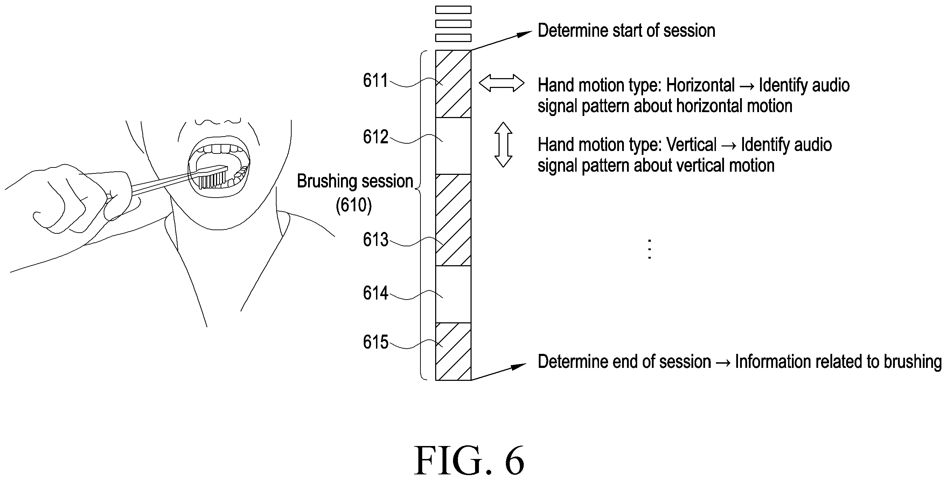

FIG. 6 is a view illustrating an example of a tooth-brushing session from the start of brushing to the end of brushing according to an embodiment.

Referring to FIG. 6, the processor 220 according to an embodiment may identify from the tooth-brushing start determination (for example, the session start determination) to the tooth-brushing end determination (for example, the session end determination) as a tooth-tooth-brushing session, and analyze motion sensing information and audio sensing information from the start of brushing to the end of brushing. The processor 220 according to an embodiment may identify, after identifying the start of the tooth-brushing session, a tooth-brushing hand motion by first motion sensing information and a first audio signal 611, a tooth-brushing hand motion by second motion sensing information and a second audio signal 612, a tooth-brushing hand motion by third motion sensing information and a third audio signal 613, a tooth-brushing hand motion by fourth motion sensing information and a fourth audio signal 614, a tooth-brushing hand motion by fifth motion sensing information and a fifth audio signal 615. According to an embodiment, the processor 220 may identify the first tooth-brushing hand motion type (e.g., horizontal motion) via the first motion sensing information, identify the first audio signal pattern type corresponding to the first tooth-brushing hand motion type, and when the first motion sensing information and the first audio signal 611 correspond to the first tooth-brushing hand motion type and the first audio signal pattern type, respectively, determine that the first motion sensing information and the first audio signal 611 correspond to the first tooth-brushing hand motion. According to an embodiment, the processor 220 may identify the second tooth-brushing hand motion type (e.g., vertical motion) via the second motion sensing information, identify the second audio signal pattern type corresponding to the second tooth-brushing hand motion type, and when the second motion sensing information and the second audio signal 612 correspond to the second tooth-brushing hand motion type and the second audio signal pattern type, respectively, determine that the second motion sensing information and the second audio signal 612 correspond to the second tooth-brushing hand motion. According to an embodiment, the processor 220 may learn motion sensing information and audio signal patterns based on past instances of tooth-brushings of the user. For example, the processor 220 may learn motion sensing information and audio signal patterns when the user brushes teeth in the horizontal direction, and motion sensing information and audio signal patterns when the user brushes teeth in the vertical direction, and may obtain, store, and then use, based on the learning, the first tooth-brushing hand motion type and the first audio signal pattern type and the second tooth-brushing hand motion type and the second audio signal pattern type. According to an embodiment, the processor 220 may respectively learn the motion sensing information and the audio signal pattern when brushing teeth in a vertical direction and when brushing teeth in a horizontal direction at respective parts of the teeth (e.g., upper tooth, lower tooth, molar, front tooth, or other parts). For example, the processor 220 may learn the motion sensing information and the audio signal pattern for each case of brushing an upper tooth in the vertical direction, brushing a lower tooth in the vertical direction, brushing a molar in the vertical direction, brushing a front tooth in the vertical direction, or brushing other parts in the vertical direction, and obtain (or store) and use, based on the learning, the first tooth-brushing hand motion type and the first audio signal pattern type corresponding to each of the upper tooth, lower tooth, molar, front tooth, or other parts. For example, the processor 220 may learn the motion sensing information and the audio signal pattern for each case of brushing the upper tooth in the vertical direction, brushing the lower tooth in the vertical direction, brushing the molar in the vertical direction, brushing the front tooth in the vertical direction, or brushing other parts in the vertical direction, and obtain (or store) and use, based on the learning, the second tooth-brushing hand motion type and the second audio signal pattern type corresponding to each of the upper tooth, lower tooth, molar, front tooth, or other parts.

According to an embodiment, the processor 220 may identify the tooth-brushing hand motion type and audio signal pattern type corresponding to the current tooth-brushing operation (or motion) of the user, and obtain the information related to the tooth-brushing operation of the user (or the information indicating which part (e.g., upper tooth, lower tooth, molar, front tooth, or other parts) of teeth and how much the user has brushed) according to the identified tooth-brushing hand motion type and audio signal pattern type.

According to an embodiment, the processor 220 may display, on the display 260, a user interface (e.g., screen) including the information indicating which part (e.g., upper tooth, lower tooth, molar, front tooth, or other parts) of teeth and how much the user has brushed. According to an embodiment, the processor 220 may transmit the information indicating which part (e.g., upper tooth, lower tooth, molar, front tooth, or other parts) of teeth and how much the user has brushed to an external electronic device (a server or an electronic device of another pre-registered user) via the communication module 219. For example, when the user of the electronic device 201 is a child, the information indicating which part (e.g., upper tooth, lower tooth, molar, front tooth, or other parts) of teeth and how much the user has brushed may be transmitted to an electronic device of the user's guardian or parent.

When the motion sensing information and the audio signal are not obtained for a predetermined period after the fifth motion sensing information and fifth audio signal 615, the processor 220 according to an embodiment may determine that the tooth-brushing is ended. In one embodiment, even when the motion sensing information and the audio signal are continuously detected, the processor 220 may still determine that the tooth-brushing is ended when the motion sensing information and the audio signal are related to another behavior pattern (e.g., walking of a user) of the user that is not tooth-brushing. The processor 220 according to an embodiment may output the information related to the tooth-brushing when it is determined that the tooth-brushing is ended or is over.

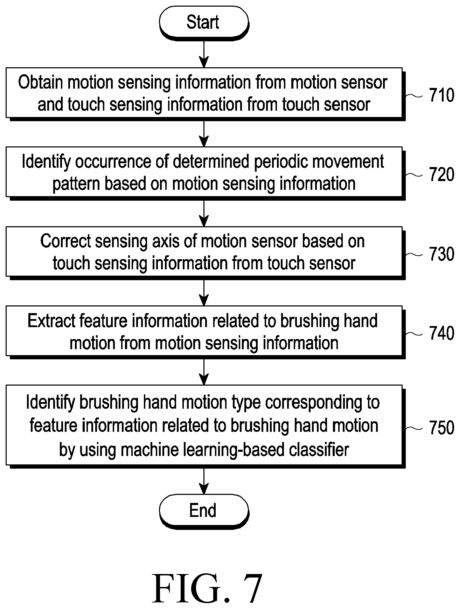

FIG. 7 is a flowchart showing a tooth-brushing hand motion type identification operation in a wearable electronic device according to an embodiment.

Referring to FIG. 7, a processor (e.g., the processor 120 in FIG. 1, the processor 220 in FIG. 2, or the processor 320 in FIG. 4, and hereinafter, the processor 220 in FIG. 2 will be described as an example.) of an electronic device (e.g., the electronic device 101 in FIG. 1, the wearable electronic device 201 in FIG. 2, the first wearable electronic device 301 in FIG. 3A, and the second wearable electronic device 302 in FIG. 3B) according to an embodiment may perform at least one of operations 710 to 750.

In operation 710, the processor 220 according to an embodiment may obtain motion sensing information from the motion sensor 212 and touch sensing information from the touch sensor 216.

In operation 720, the processor 220 according to an embodiment may identify the occurrence of the designated periodic motion pattern based on the motion sensing information from the motion sensor 212. For example, the designated periodic motion pattern may be a pattern obtained by learning previous motion patterns during tooth-brushing. For example, the processor 220 according to an embodiment may identify that the tooth-brushing has started when the designated motion pattern occurs.

In operation 730, the processor 220 according to an embodiment may correct, based on the touch sensing information obtained from the touch sensor 216, sensing axes of the motion sensor 212. For example, the processor 220 according to an embodiment may correct at least one sensing axis of the acceleration sensor, the gyro sensor, and/or the geomagnetic sensor included in the motion sensor 212. For example, when the electronic device 201 is the second ring-shaped wearable device 302, there is a high possibility that the second wearable device 302 rotates around the finger and it may be difficult to accurately identify the posture of the second wearable device 302 in relation to the user due to the frequent changes in the posture of the second wearable device 302. Therefore, when the second wearable device 302 is ring-shaped, the sensing axis of the motion sensor 212 may be corrected via the touch sensor 216.

For example, when the electronic device 201 is the second ring-shaped wearable device 302, the wearing state and the wearing position of the second wearable device 302 may be detected, and the sensing axis of the motion sensor 212 may be corrected based thereon. For example, based on the touch sensing information sensed via the touch sensor, when the second wearable device 302 is worn on a finger, the processor 220 may identify how much it is rotated with respect to a preset wearing reference point and configure, based on the rotation state, a rotation matrix to rotate and correct each axes of the motion sensors 212 so as to correct axis information.

In operation 740, the processor 220 according to an embodiment may extract feature information related to the tooth-brushing hand motion from the motion sensing information. For example, the processor 220 may obtain (or extract) information about the hand posture, the frequency characteristics with respect to the hand motion, and the magnitude and direction of the hand motion, which are obtained via the acceleration value sensed by the acceleration sensor, the rotation direction or rotation angle value of the electronic device 201 sensed by the gyro sensor, and geomagnetic direction information sensed by the geomagnetic sensor. For example, the hand posture related to tooth-brushing may be a posture in which the back of the hand faces upward (toward the sky) with reference to the body or faces to the left or right with reference to the body. For example, the magnitude of the hand motion related to tooth-brushing may vary according to the speed of tooth-brushing, but may be lower than or equal to a designated size. For example, the direction of the hand motion related to tooth-brushing may be the horizontal direction (or forward and backward directions or left and right directions) with reference to the body or the vertical direction (or upward and downward directions) with reference to the body. For example, the direction of the hand motion related to tooth-brushing may include direction information in a case of moving in a circle while including a horizontal direction and the vertical direction. For example, the frequency characteristics with respect to the hand motion related to tooth-brushing may include a designated frequency component (e.g., 10 Hz or higher frequency components). The designated frequency component may be a high frequency signal component generated when the toothbrush hits the gums and the upper or lower side of the oral cavity during brushing.

In operation 750, the processor 220 according to an embodiment may identify the tooth-brushing hand motion type corresponding to the feature information related to the tooth-brushing hand motion by using a machine learning-based classifier. For example, by using the machine learning-based classifier, the processor 220 may compare at least a piece of pre-obtained tooth-brushing hand motion information (or at least one tooth-brushing hand motion pattern) (e.g., the first tooth-brushing hand motion information (or pattern) and/or the second tooth-brushing hand motion information (or pattern)) and the feature information related to the tooth-brushing hand motion, and identify whether the feature information related to the tooth-brushing hand motion corresponds to the first tooth-brushing hand motion pattern or the second tooth-brushing hand motion pattern. The processor 220 according to an embodiment may determine (confirm or identify) the tooth-brushing hand motion type corresponding to the first motion sensing information as the first tooth-brushing hand motion when the first motion sensing information corresponds to the first tooth-brushing hand motion pattern. The processor 220 according to an embodiment may determine (confirm or identify) the tooth-brushing hand motion type corresponding to the first motion sensing information as the second tooth-brushing hand motion when the first motion sensing information corresponds to the second tooth-brushing hand motion pattern.

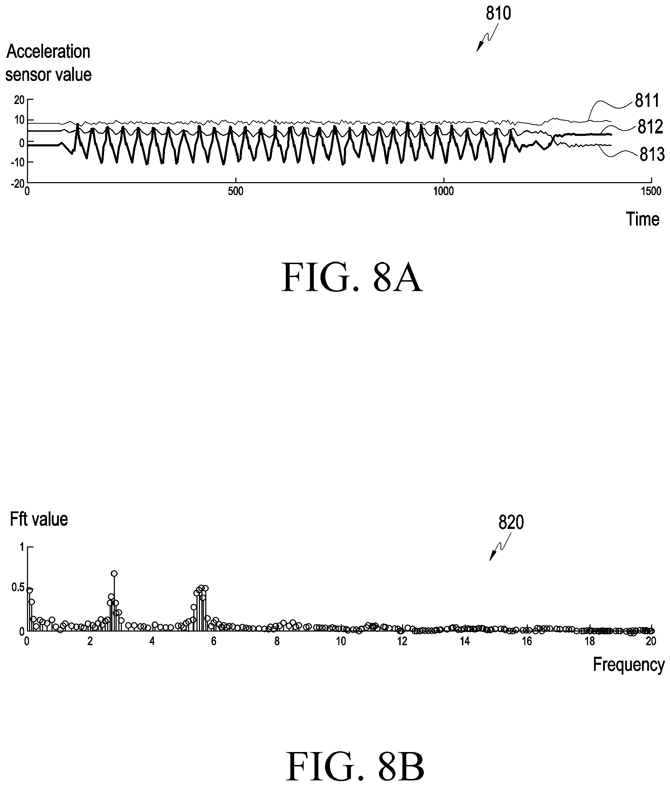

FIG. 8A is a view illustrating an example of a first acceleration sensor data graph when brushing teeth in the horizontal direction according to an embodiment, and FIG. 8B is a view illustrating a fast Fourier transform (FFT) graph with respect to the first acceleration sensor data when brushing teeth in the horizontal direction according to an embodiment.

Referring to FIG. 8A, when brushing teeth in the horizontal direction, the processor 220 may obtain a first acceleration sensor data graph 810. In the first acceleration graph 810, the horizontal axis may indicate time (ms) and the vertical axis may indicate acceleration sensor value (m/s²). According to an embodiment, when brushing teeth in the horizontal direction, the first acceleration sensor data graph 810 may include a z-axis acceleration sensor data curve 811, a y-axis acceleration sensor data curve 812, and an x-axis acceleration sensor data curve 813.

Referring to FIG. 8B, the processor 220 according to an embodiment may perform frequency analysis on the first acceleration sensor data graph 810 via fast Fourier transform (FFT) to obtain a first FFT graph 820. In the first FFT graph 820, the horizontal axis may indicate frequency (Hz) and the vertical axis may indicate FFT value. According to the first FFT graph 820, when brushing teeth in a horizontal direction, a low-frequency component under a first frequency (e.g., about 6 Hz) may be mainly included.

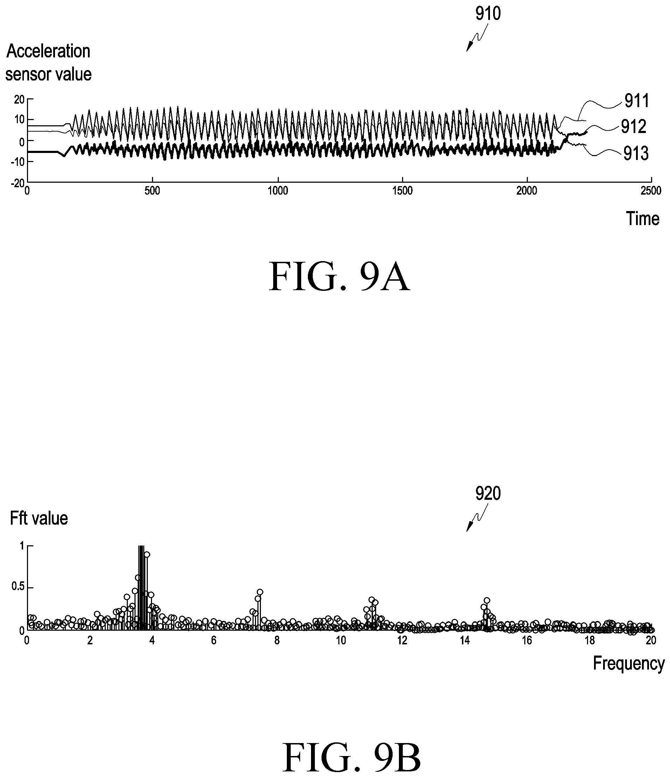

FIG. 9A is a view illustrating an example of a second acceleration sensor data graph when brushing teeth in the vertical direction according to an embodiment, and FIG. 9B is a view illustrating an FFT transform graph with respect to the second acceleration sensor data when brushing teeth in the vertical direction according to an embodiment.

Referring to FIG. 9A, when brushing teeth in the vertical direction according to an embodiment, the processor 220 may obtain a second acceleration sensor data graph 910. In the second acceleration graph 910, the horizontal axis may indicate time (ms) and the vertical axis may indicate acceleration sensor value (m/s²). According to an embodiment, when brushing teeth in the vertical direction, the second acceleration sensor data graph 910 may include a z-axis acceleration sensor data curve 911, a y-axis acceleration sensor data curve 912, and an x-axis acceleration sensor data curve 913.

Referring to FIG. 9B, the processor 220 according to an embodiment may perform frequency analysis on the second acceleration sensor data graph 910 via fast Fourier transform (FFT) to obtain a second FFT graph 920. In the second FFT graph 920, the horizontal axis may indicate frequency (Hz) and the vertical axis may indicate FFT value. According to the second FFT graph 920, when brushing teeth in the vertical direction, a high-frequency component higher than or equal to a second frequency (e.g., about 10 Hz) may be included. For example, the high-frequency component may be a frequency component which occurs when the toothbrush hits the gums and the upper or lower side of the oral cavity during brushing.

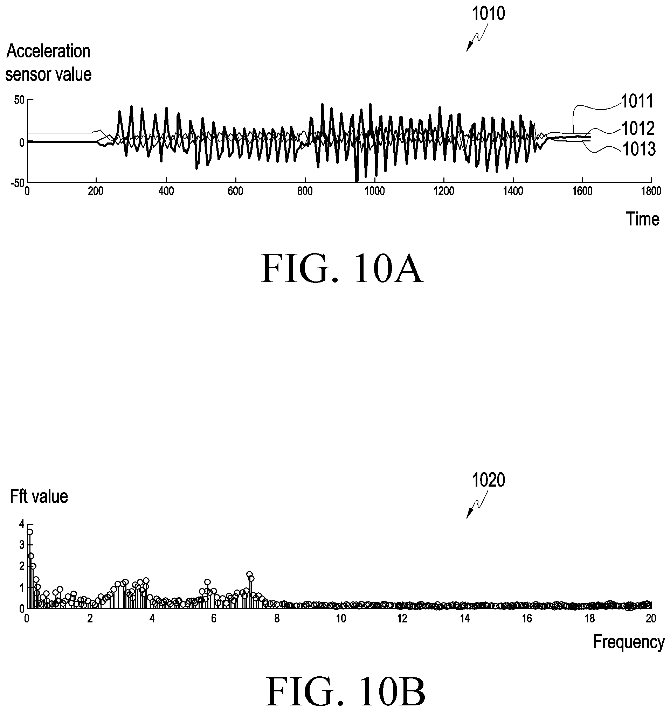

FIG. 10A is a view illustrating an example of a third acceleration sensor data graph when the user is drying hair according to an embodiment, and FIG. 10B is a view illustrating an FFT transform graph with respect to the third acceleration sensor data when drying hair according to an embodiment.

Referring to FIG. 10A, when the user is drying hair according to an embodiment, the processor 220 may obtain a third acceleration sensor data graph 1010. In the third acceleration graph 1010, the horizontal axis may indicate time (ms) and the vertical axis may indicate acceleration sensor value (m/s²). According to an embodiment, when drying hair, the third acceleration sensor data graph 1010 may include a z-axis acceleration sensor data curve 1011, a y-axis acceleration sensor data curve 1012, and an x-axis acceleration sensor data curve 1013.

Referring to FIG. 10B, the processor 220 according to an embodiment may perform frequency analysis on the third acceleration sensor data graph 1010 via fast Fourier transform (FFT) to obtain a third FFT graph 1020. In the third FFT graph 1020, the horizontal axis may indicate frequency (Hz) and the vertical axis may indicate FFT value. According to the third FFT graph 1020, since the frequency components mainly appear in the low frequency band of 8 Hz or less, similar to brushing teeth in the horizontal direction, but the size of motion due to the hand motion appears relatively larger than when brushing teeth, the processor 220 may not identify the hair drying motion as tooth-tooth-brushing hand motion.

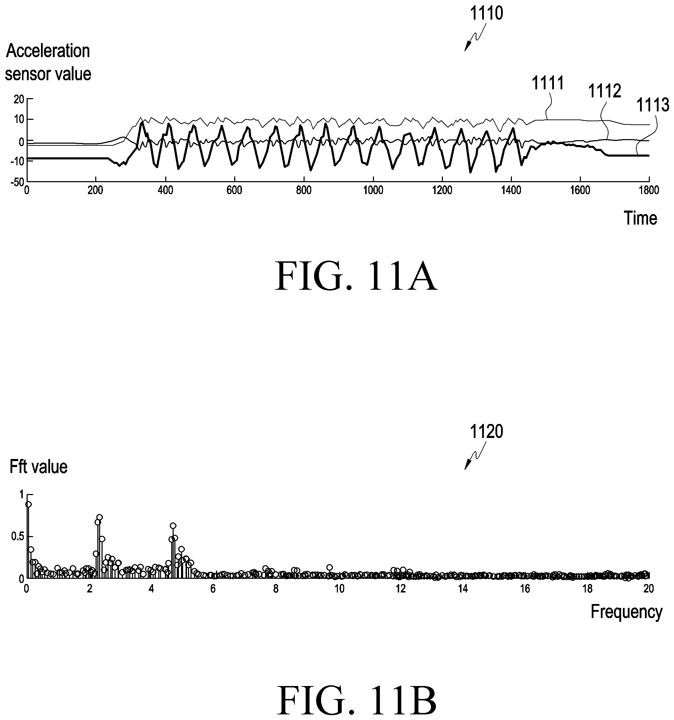

FIG. 11A is a view illustrating an example of a fourth acceleration sensor data graph when the user is washing hands according to an embodiment, and FIG. 11B is a view illustrating an FFT transform graph with respect to the fourth acceleration sensor data when washing hands according to an embodiment.

Referring to FIG. 11A, when the user is washing hands according to an embodiment, the processor 220 may obtain a fourth acceleration sensor data graph 1110. In the fourth acceleration graph 1110, the horizontal axis may indicate time (ms) and the vertical axis may indicate acceleration sensor value (m/s²). According to an embodiment, when washing hands, the fourth acceleration sensor data graph 1110 may include a z-axis acceleration sensor data curve 1111, a x axis acceleration sensor data curve 1112, and an y axis acceleration sensor data curve 1113.

Referring to FIG. 11B, the processor 220 according to an embodiment may perform frequency analysis on the fourth acceleration sensor data graph 1110 via fast Fourier transform (FFT) to obtain a fourth FFT graph 1120. In the fourth FFT graph 1120, the horizontal axis may indicate frequency (Hz) and the vertical axis may indicate FFT value. According to the fourth FFT graph 1120, the frequency components mainly appear in the low frequency band of 8 Hz or less, similar to brushing teeth in the horizontal direction, and the size of motion due to the hand motion may not be much different from when brushing teeth, and in this case, the processor 220 may not identify the hand washing motion as the tooth-tooth-brushing hand motion by further taking into consideration the hand posture information or the hand motion direction information.

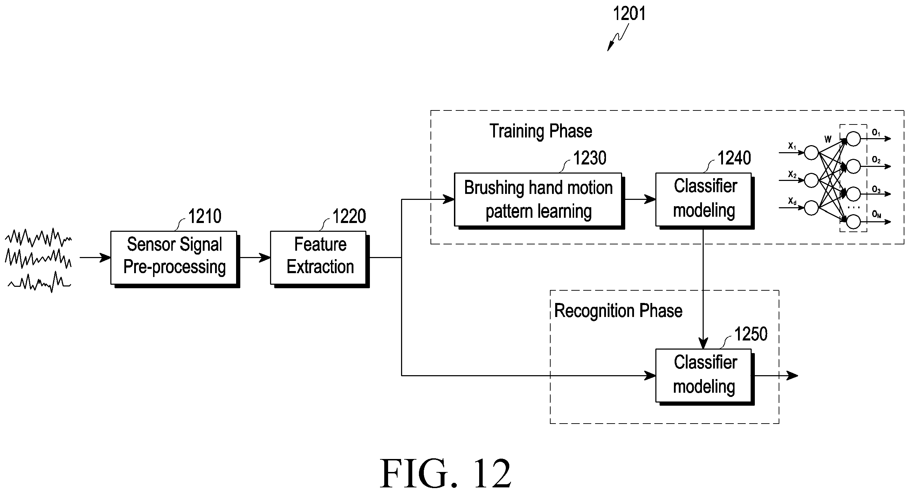

FIG. 12 is a view for illustrating a machine learning-based classifier model according to an embodiment.

Referring to FIG. 12, a machine learning-based classifier model 1201 according to an embodiment may be a software module executed by the processor 220 or a hardware module including at least one electrical circuit. For example, the machine learning-based classifier model 1201 may include at least a part or all of a sensor signal pre-processing unit 1210, a feature extraction unit 1220, a tooth-brushing hand motion pattern learning unit 1230, a classifier modeling unit 1240, and a classifier model 1250. According to an embodiment, each of the sensor signal pre-processing unit 1210, the feature extraction unit 1220, the tooth-brushing hand motion pattern learning unit 1230, the classifier modeling unit 1240, and the classifier model 1250 may be a software program executed by the processor 220 or hardware including at least one electrical circuit.

The sensor signal pre-processing unit 1210 according to an embodiment may pre-process the sensor signal obtained by the motion sensor 212 to obtain the sensor data. For example, the sensor signal pre-processing unit 1210 may obtain the acceleration and gyro sensor data from the sensor signal sensed via the motion sensor 212, correct the sensor axis, and perform filtering for removing noise.

The feature extraction unit 1220 according to an embodiment may extract feature information from the acceleration and gyro sensor data. For example, the hand posture information, the frequency characteristic information with respect to the tooth-brushing hand motion, and the information about the magnitude and direction of the movement of the tooth-brushing hand motion may be obtained from the acceleration and gyro sensor data.

The tooth-brushing hand motion pattern learning unit 1230 according to an embodiment may learn, based on the extracted feature information, the tooth-brushing hand motion pattern via a machine learning method.

The classifier modeling unit 1240 according to an embodiment may model, based on the result of the tooth-brushing hand motion learning, a classifier configured to classify the tooth-brushing hand motion according to types thereof, and modeling information may be provided by the classification.

The classifier model 1250 according to an embodiment may identify the tooth-brushing hand motion type corresponding to the extracted feature information based on the feature information extracted from the feature extraction unit 1220 and the classifier modeling information provided from the classifier modeling unit 1240. For example, the classifier modeling unit 1240 may identify whether the extracted feature information corresponds to the first tooth-brushing motion pattern or the second tooth-brushing motion pattern, or may identify whether the extracted feature information corresponds to the hand motion pattern other than the tooth-tooth-brushing motion pattern. For example, the other hand motion patterns may include a running motion, a hair drying motion, or a hand washing motion. For example, the classifier modeling unit 1240 may determine that the tooth-brushing motion is detected when the extracted feature information includes the first tooth-brushing motion pattern or the second tooth-brushing motion pattern.

According to an embodiment, the operations of the feature extraction unit 1220 and the tooth-brushing hand motion pattern learning unit 1230 may be in a pre-training phase which may be performed by an external electronic device or an external server instead of the processor 220 of the electronic device 201. When the operations of the feature extraction unit 1220 and the tooth-brushing hand motion pattern learning unit 1230 are performed by another device, the learning result of the pre-training may be provided to the electronic device 201 and the electronic device 201 may perform a recognition phase using the learning result of the pre-training and the classifier model 1250.

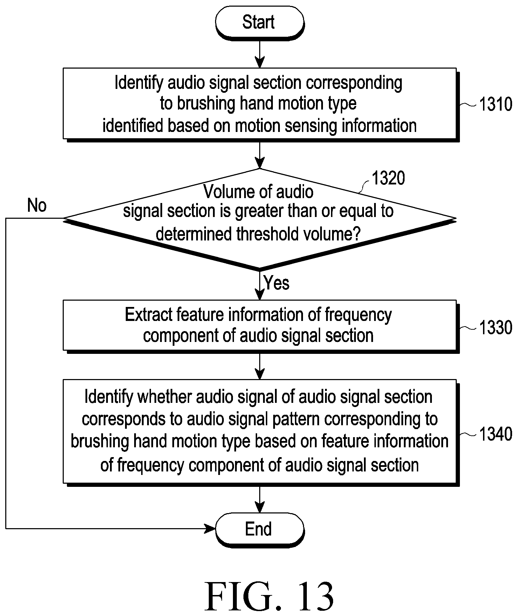

FIG. 13 is a flowchart showing a tooth-brushing sound identification operation using an audio signal pattern type corresponding to a tooth-brushing hand motion type in a wearable electronic device according to an embodiment.

Referring to FIG. 13, a processor (e.g., the processor 120 in FIG. 1, the processor 220 in FIG. 2, or the processor 320 in FIG. 4, and hereinafter, the processor 220 in FIG. 2 will be described as an example.) of an electronic device (e.g., the electronic device 101 in FIG. 1, the wearable electronic device 201 in FIG. 2, the first wearable electronic device 301 in FIG. 3A, and the second wearable electronic device 302 in FIG. 3B) according to an embodiment may perform at least one of operations 1310 to 1340.

In operation 1310, the processor 220 according to an embodiment may identify an audio signal section corresponding to the tooth-brushing hand motion type identified based on the motion sensing information. For example, the processor 220 may distinguish (e.g., segmentation) and identify the audio signal section (e.g., a first audio signal section and a second audio signal section) corresponding to each tooth-brushing hand motion type based on to the identified tooth-brushing hand motion type (e.g., the first tooth-brushing hand motion type or the second tooth-brushing hand motion type) among all the audio signal sections.

In operation 1320, the processor 220 according to an embodiment may confirm whether the volume of the audio signal section corresponding to the tooth-brushing hand motion type is equal to or greater than a designated threshold volume. For example, the threshold volume may be specified with various values within the range in which the tooth-brushing sound can be recognized. For example, when the user closes his/her mouth and brushes his/her teeth while brushing his/her teeth, since the volume of the tooth-brushing sound is extremely low and it is impossible to recognize the tooth-brushing sound, it may be necessary to set the threshold volume within the range where the tooth-brushing sound can be recognized. The processor 220 according to an embodiment may end the processing of the audio signal section when the volume of the audio signal section is not greater than or equal to the designated volume. The processor 220 according to an embodiment may perform operation 1330 when the volume of the audio signal section is greater than or equal to the designated volume.

In operation 1330, the processor 220 according to an embodiment may extract feature information of a frequency component of the audio signal section corresponding to the tooth-brushing hand motion type. For example, the processor 220 converts an audio signal in the time domain into an audio signal in the frequency domain via the FFT, and extract the feature information (e.g., a local maximum value (local maxima) with respect to a single-sided amplitude spectrum) of the frequency component from the audio signal in the frequency domain.

In operation 1340, the processor 220 according to an embodiment may identify whether the audio signal in the audio signal section corresponds to an audio signal pattern corresponding to the tooth-brushing hand motion type, based on the feature information of the frequency component of the audio signal section corresponding to the tooth-brushing hand motion type. For example, the processor 220 may identify whether the frequency component of the audio signal section corresponding to the first tooth-brushing hand motion type corresponds to the first audio signal pattern corresponding to the first tooth-brushing hand motion type, or identify whether the frequency component of the audio signal section corresponding to the second tooth-brushing hand motion type corresponds to the second audio signal pattern corresponding to the second tooth-brushing hand motion type. For example, the processor 220 may previously learn the feature information of the frequency component of the audio signal section corresponding to the tooth-brushing hand motion type and may obtain the audio signal pattern analysis model based on the result of learning. The processor 220 according to an embodiment may identify whether the audio signal in the audio signal section corresponding to the tooth-brushing hand motion type corresponds to the audio signal pattern corresponding to the tooth-brushing hand motion type via the audio signal pattern analysis model. When the audio signal in the audio signal section corresponding to the tooth-brushing hand motion type corresponds to the audio signal pattern corresponding to the tooth-brushing hand motion type, the processor 220 according to an embodiment may determine (confirm or identify) determine the audio signal as the tooth-brushing sound corresponding to the tooth-brushing hand motion type.

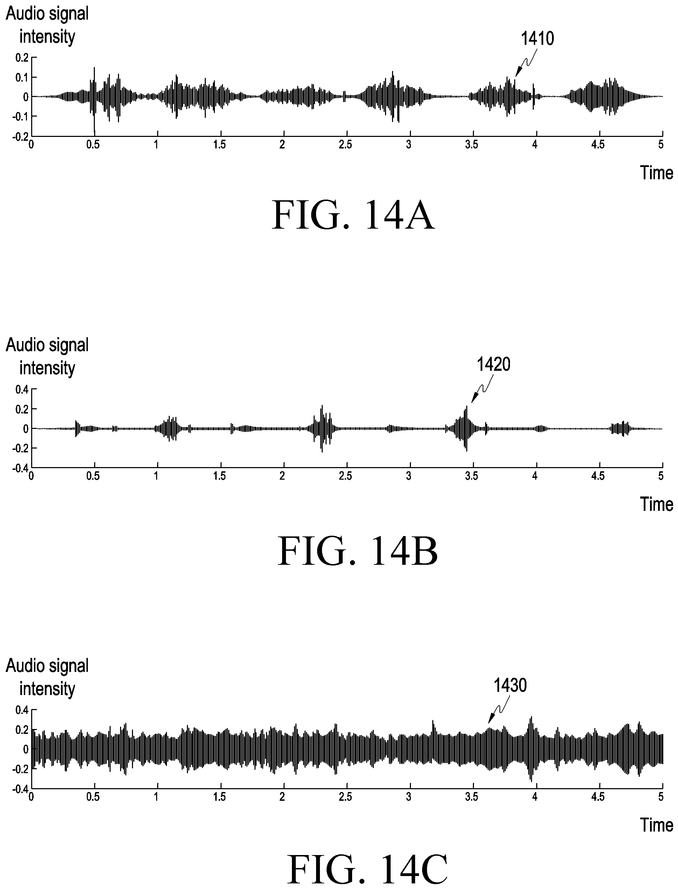

FIG. 14A is a view illustrating an example of an audio signal graph obtained when the user is brushing teeth in the horizontal direction according to an embodiment, FIG. 14B is a view illustrating an example of an audio signal graph obtained when the user is brushing teeth in the vertical direction according to an embodiment, and FIG. 14C is a view illustrating an example of an audio signal graph obtained when the user is washing hands according to an embodiment.

Referring to FIG. 14A, when the user is brushing teeth in the horizontal direction according to an embodiment, the processor 220 may obtain a first audio signal graph 1410. In the first audio signal graph 1410, the horizontal axis may indicate time (ms) and the vertical axis may indicate audio signal amplitude. According to an embodiment, when the user is brushing teeth in the horizontal direction, the teeth-brushing may make sound caused by friction between the toothbrush and teeth, and since the time of friction between the toothbrush and teeth is longer than when brushing teeth in the vertical direction, the audio signal may be generated at longer time intervals (e.g., first time intervals).

Referring to FIG. 14B, when the user is brushing teeth in the vertical direction according to an embodiment, the processor 220 may obtain a second audio signal graph 1420. In the second audio signal graph 1420, the horizontal axis may indicate time (ms) and the vertical axis may indicate audio signal amplitude. According to an embodiment, when the user is brushing teeth in the vertical direction, the teeth-brushing may make sound when the toothbrush is caught by the gums or hits the upper or lower side of the oral cavity, and since the time of friction between the toothbrush and teeth is shorter than when brushing teeth in the horizontal direction, the audio signal may be generated at shorter time intervals (e.g., second time intervals).

Referring to FIG. 14C, when motion (e.g., washing hands) other than brushing teeth occurs according to an embodiment, the processor 220 may obtain a third audio signal graph 1430. In the third audio signal graph 1430, the horizontal axis may indicate time (ms) and the vertical axis may indicate audio signal amplitude. According to an embodiment, regardless of the motion of washing hands during the hand washing, the audio signal about the sound of water may be input, and thus an audio signal with a quite different pattern from that in a case of brushing in the horizontal direction or brushing in the vertical direction may be generated.

According to an embodiment the processor 220 of the electronic device 201 may learn various previous audio signal patterns according to various hand motions to obtain an audio signal pattern according to the type of tooth-brushing hand motion. The electronic device 201 may be implemented to obtain audio signal pattern information according to the type of tooth-brushing hand motion pre-learned from an external electronic device (or a server) via communication and store it in a memory 230, or during the manufacturing, load (or store) the audio signal pattern information according to the pre-learned type of tooth-brushing hand motion to the memory 230.

According to an embodiment, various modeling methods may be used when obtaining the audio signal pattern according to the type of tooth-brushing motion. For example, the processor 220 may obtain the audio signal pattern according to the tooth-brushing hand motion type by using a Gaussian mixture model-hidden Markov model (GMM-HMM) mixed model.

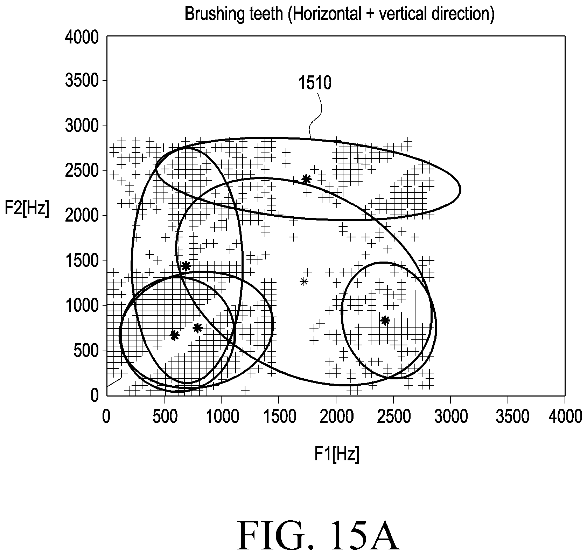

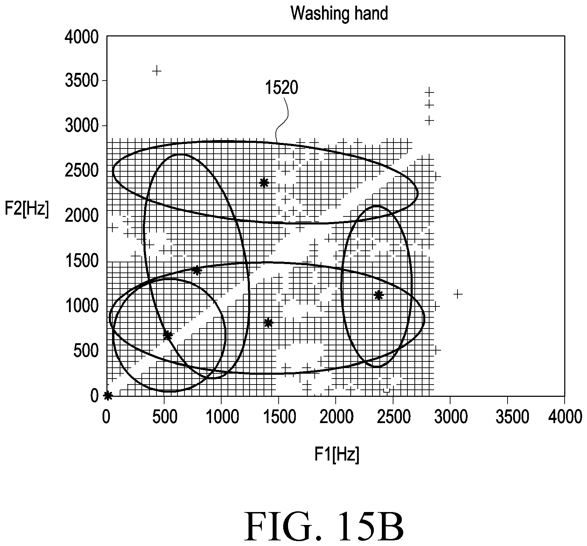

FIG. 15A is a graph showing the result of learning, by using a GMM model, two best frequency components in various local maxima frequency features in a single-sided amplitude spectrum in an audio signal during tooth-brushing in which the motion in the horizontal direction and the motion in the vertical direction are mixed according to an embodiment, and FIG. 15B is a graph showing the result of learning, by using a GMM model, two best frequency components in various local maxima frequency features in a single-sided amplitude spectrum in an audio signal when washing hands according to an embodiment.

Referring to FIG. 15A and FIG. 15B, the horizontal axis may indicate a first frequency component F1 and the vertical axis may indicate a second frequency component F2. Looking at the feature distribution indicated by "+", the feature distribution (+) during tooth-brushing in which the horizontal motion and the vertical motion are mixed as in FIG. 15A may be similar to the feature distribution (+) during hand washing as in FIG. 15B. In FIG. 15A and FIG. 15B, the learning results 1510 and 1520 of the six GMM models may appear similarly to each other, respectively. This can be considered that the features or states observed in the audio signals are similar, and when analyzing time-series patterns via HMM with this similar information, it will ultimately raise the concern that misrecognition will occur in distinguishing between tooth-brushing and hand-washing sounds. Therefore, when using the frequency feature distribution during tooth-brushing in which horizontal and vertical motions are mixed, it is difficult to distinguish from other hand motions (e.g., hand washing), and thus an error may occur in detecting the tooth-brushing.

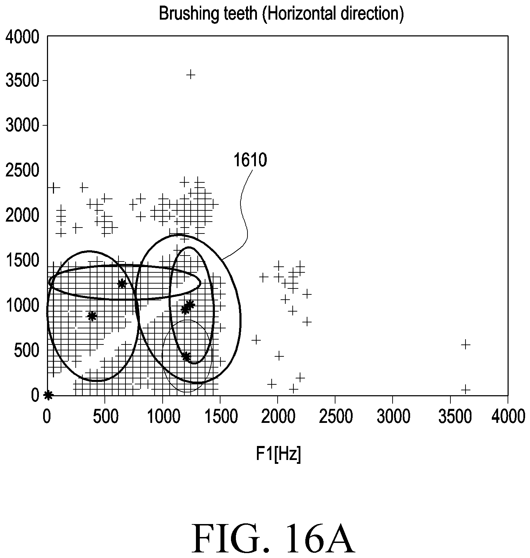

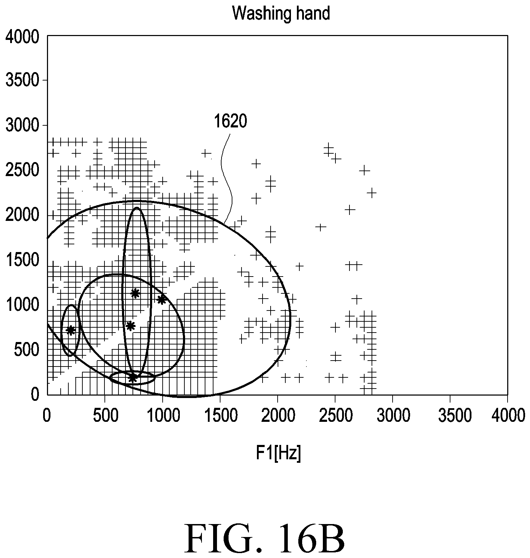

FIG. 16A is a graph showing the result of learning, by using a GMM model, two best frequency components in various local maxima frequency features in a single-sided amplitude spectrum in an audio signal when brushing teeth in the horizontal direction according to an embodiment, and FIG. 16B is a graph showing the result of learning, by using a GMM model, two best frequency components in various local maxima frequency features in a single-sided amplitude spectrum in an audio signal when brushing teeth in the vertical direction according to an embodiment.

Referring to FIG. 16A and FIG. 16B, the horizontal axis may indicate a first frequency component F1 and the vertical axis may indicate a second frequency component F2. Looking at the feature distribution indicated by "+", the feature distribution (+) during tooth-brushing in the horizontal direction as in FIG. 16A and the feature distribution (+) during tooth-brushing in the vertical direction as in FIG. 16B may each be different from the feature distribution (+) during hand washing as in FIG. 15B.

The learning results 1610 and 1620 of the six GMM models in FIG. 16A and FIG. 16B may appear quite different from the learning result 1520 of the six GMM models in FIG. 15B, respectively. Therefore, as described in an embodiments of the disclosure, when the horizontal and vertical motions are recognized separately and the frequency feature distribution is used, it is easy to distinguish them from other hand motions (e.g., hand washing), and thus it can reduce errors in tooth-brushing detection than when using the frequency feature distribution during tooth-brushing in which horizontal and vertical motions are mixed.

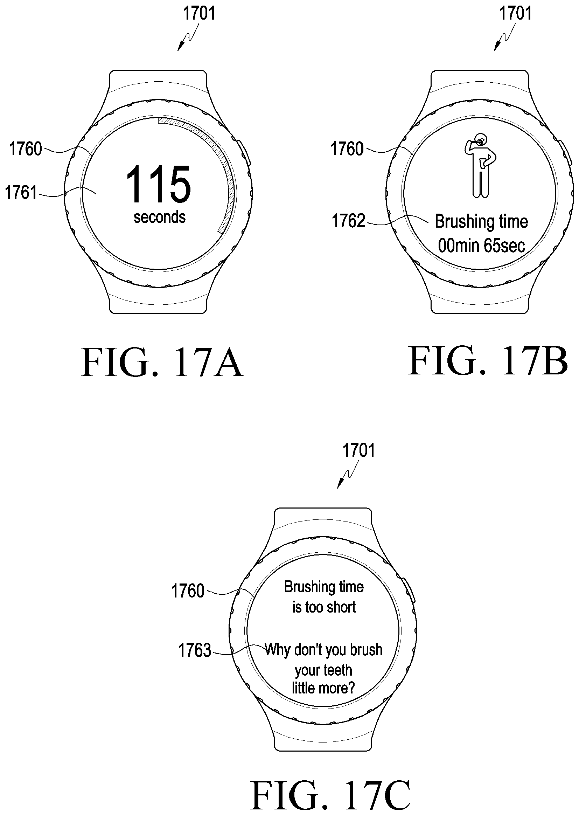

FIG. 17A is a view illustrating an example of a first information display related to tooth-brushing in a wearable electronic device according to an embodiment.

Referring to FIG. 17A, a processor (e.g. the processor 120 in FIG. 1 or the processor 220 in FIG. 2, and hereinafter, the processor 220 in FIG. 2 will be described as an example.) of a wearable electronic device 1701 (e.g., the electronic device 101 in FIG. 1, the wearable electronic device 201 in FIG. 2, and the first wearable electronic device 301 in FIG. 3A) according to an embodiment may control a display 1760 (e.g., the display module 160 in FIG. 1 or the display 260 in FIG. 2) to display first information 1761 related to tooth-brushing. For example, the first information 1761 related to the tooth-brushing may include tooth-brushing time counting information (e.g., 115 seconds) that is counted after the user starts brushing teeth.

FIG. 17B is a view illustrating an example of a second information display related to tooth-brushing in a wearable electronic device according to an embodiment.

Referring to FIG. 17B, the processor 220 according to an embodiment may control the display 1760 to display second information 1762 related to tooth-brushing. For example, the second information 1762 related to the tooth-brushing may include total tooth-brushing time (e.g., tooth-brushing time for 65 seconds) from the start of brushing to the end of brushing of a user.

FIG. 17C is a view illustrating an example of a third information display related to tooth-brushing in a wearable electronic device according to an embodiment.

Referring to FIG. 17C, the processor 220 according to an embodiment may control the display 1760 to display third information 1763 related to tooth-brushing. For example, the third information 1763 related to tooth-brushing may include a message (e.g., "Brushing time is too short. Why don't you brush your teeth a little more?") informing the user that the total tooth-brushing time from the start to the end of brushing is shorter than the recommended time.

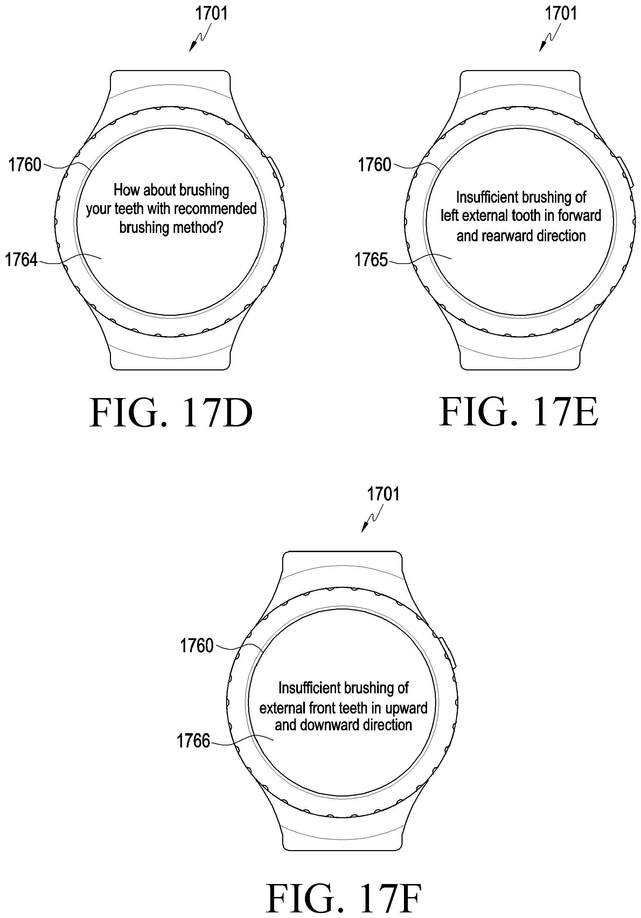

FIG. 17D is a view illustrating an example of a fourth information display related to tooth-brushing in a wearable electronic device according to an embodiment.

Referring to FIG. 17D, the processor 220 according to an embodiment may control the display 1760 to display fourth information 1764 related to tooth-brushing. For example, the fourth information 1764 related to tooth-brushing may include a message (e.g., "How about brushing your teeth with the recommended tooth-brushing method?" or "Tooth-brushing was not performed according to the recommended tooth-brushing method.") informing the user whether the user has followed the recommended brushing method identified based on the number of times of performing the tooth-brushing hand motion type by the user.

Referring to FIG. 17E, the processor 220 according to an embodiment may control the display 1760 to display fifth information 1765 related to tooth-brushing. For example, the fifth information 1765 related to tooth-brushing may include a message (e.g., "Brushing in left and right directions on the left outer tooth is insufficient." or "Why don't you brush your teeth more in left and right directions?") indicating that the first tooth-brushing hand motion is insufficient in the tooth-brushing, based on the type of tooth-brushing hand motion performed by the user.

Referring to FIG. 17F, the processor 220 according to an embodiment may control the display 1760 to display sixth information 1766 related to tooth-brushing. For example, the sixth information 1766 related to tooth-brushing may include a message (e.g., "Brushing in upward and downward directions on the front outer tooth is insufficient." or "Why don't you brush your teeth more in upward and downward directions?") indicating that the second tooth-brushing hand motion is insufficient in the tooth-brushing, based on the type of tooth-brushing hand motion performed by the user.

According to certain embodiments, it may be apparent to those skilled in the art that in addition to the examples shown in FIGS. 17A to 17F, other information related to tooth-brushing can be further displayed.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B", "at least one of A and B", "at least one of A or B", "A, B, or C", "at least one of A, B, and C", and "at least one of A, B, or C", may include any one of, or all possible combinations of the items enumerated together in a corresponding one of the phrases. As used herein, such terms as "1st" and "2nd", or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with the disclosure, the term "module" may include a unit implemented in hardware, software, or firmware, and may interchangeably be used with other terms, for example, "logic", "logic block", "part", or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to an embodiment, in a non-transitory storage medium storing instructions, wherein the instructions are configured to enable, when executed by at least one processor of a wearable electronic device, the wearable electronic device to perform at least one operation including obtaining motion sensing information via a motion sensor, obtaining an audio signal corresponding to the motion sensing information via an audio sensor, identifying a tooth-brushing hand motion type corresponding to the motion sensing information, identifying an audio signal pattern corresponding to the tooth-brushing hand motion type, and identifying a tooth-brushing hand motion corresponding to the motion sensing information and the audio signal, based on the tooth-brushing hand motion type and the audio signal pattern.

The embodiments disclosed in the specification and drawings are merely provided for specific examples to easily explain the technical content according to the embodiments of the disclosure and help the understanding of the embodiments of the disclosure, and are not intended to limit the scope of the embodiments of the disclosure. Therefore, the scope of the various embodiments of the disclosure should be construed that all changes or modified forms derived based on the technical idea of various embodiments of the disclosure in addition to the embodiments disclosed herein are included in the scope of the various embodiments of the disclosure.

## Claims

1. A wearable electronic device (201) comprising:
a motion sensor (212);
an audio sensor (218);
a display (260);
a memory (230); and
a processor (220) electrically connected to the motion sensor, the audio sensor, and the memory,
wherein the processor is configured to:
obtain motion sensing information via the motion sensor,
obtain an audio signal corresponding to the motion sensing information via the audio sensor,
identify a tooth-brushing hand motion type corresponding to the motion sensing information,
identify an audio signal pattern corresponding to the tooth-brushing hand motion type, and
identify, based on the tooth-brushing hand motion type and the audio signal pattern, a tooth-brushing hand motion corresponding to the motion sensing information and the audio signal.

2. The wearable electronic device of claim 1, wherein the tooth-brushing hand motion type includes a first tooth-brushing hand motion type and a second tooth-brushing hand motion type, and
wherein the processor is configured to:
identify a first audio signal pattern corresponding to the first tooth-brushing hand motion type when the tooth-brushing hand motion type is the first tooth-brushing hand motion type, and
identify a second audio signal pattern corresponding to the second tooth-brushing hand motion type when the tooth-brushing hand motion type is the second tooth-brushing hand motion type,
wherein the first tooth-brushing hand motion type indicates a tooth-brushing hand motion type in a horizontal direction, and the second tooth-brushing hand motion type indicates a tooth-brushing hand motion type in a vertical direction.

3. The wearable electronic device of claim 1 or claim 2, wherein the motion sensor includes an acceleration sensor, a gyro sensor, and a geomagnetic sensor, and
wherein the motion sensing information includes at least one of hand posture information, frequency characteristic information with respect to the tooth-brushing hand motion, and information about magnitude and direction of the tooth-brushing hand motion which are obtained based on an acceleration value sensed by the acceleration sensor, a rotation direction or rotation angle value of the electronic device sensed by the gyro sensor, and geomagnetic direction information sensed by the geomagnetic sensor.

4. The wearable electronic device of any one of claim 1 to claim 3, further comprising a touch sensor (216),
wherein the processor is configured to:
identify a start of tooth-brushing when a designated periodic motion pattern is identified based on the motion sensing information, and
correct, based on touch sensing information obtained by the touch sensor, a sensing axis of the motion sensor.

5. The wearable electronic device of any one of claim 1 to claim 4, wherein the processor is configured to:
identify an end of tooth-brushing when the motion sensing information and/or the audio signal are not obtained for a designated time period, and
control the display to display, based on the identification of the end of tooth-brushing, information related to tooth-brushing from the start of tooth-brushing to the end of tooth-brushing.

6. The wearable electronic device of any one of claim 1 to claim 5, further comprising a communication module (219),
wherein the processor is configured to transmit the information related to tooth-brushing to an external electronic device via the communication module.

7. The wearable electronic device of any one of claim 1 to claim 6, wherein the processor is configured to compare tooth-brushing hand motion type information previously obtained by learning using a machine learning-based classifier model with feature information extracted from the motion sensing information to identify the tooth-brushing hand motion type.

8. A method for providing tooth-brushing information in a wearable electronic device (201), the method comprising:
obtaining motion sensing information via a motion sensor (212) of the wearable electronic device;
obtaining an audio signal corresponding to the motion sensing information via an audio sensor (218) of the wearable electronic device;
identifying a tooth-brushing hand motion type corresponding to the motion sensing information;
identifying an audio signal pattern corresponding to the tooth-brushing hand motion type; and
identifying, based on the tooth-brushing hand motion type and the audio signal pattern, a tooth-brushing hand motion corresponding to the motion sensing information and the audio signal.

9. The method of claim 8, wherein the tooth-brushing hand motion type includes a first tooth-brushing hand motion type and a second tooth-brushing hand motion type,
the method further comprising:
identifying a first audio signal pattern corresponding to the first tooth-brushing hand motion type when the tooth-brushing hand motion type is the first tooth-brushing hand motion type; and
identifying a second audio signal pattern corresponding to the second tooth-brushing hand motion type when the tooth-brushing hand motion type is the second tooth-brushing hand motion type,
wherein the first tooth-brushing hand motion type indicates tooth-brushing hand motion type in a horizontal direction, and
wherein the second tooth-brushing hand motion type indicates tooth-brushing hand motion type in a vertical direction.

10. The method of claim 8 or claim 9, wherein the motion sensor includes an acceleration sensor, a gyro sensor, and a geomagnetic sensor, and
the motion sensing information includes at least one of hand posture information, frequency characteristic information with respect to the tooth-brushing hand motion, and information about magnitude and direction of the tooth-brushing hand motion which are obtained based on an acceleration value sensed by the acceleration sensor, a rotation direction or rotation angle value of the electronic device sensed by the gyro sensor, and geomagnetic direction information sensed by the geomagnetic sensor.

11. The method of any one of claim 8 to claim 10, further comprising:
identifying a start of tooth-brushing when a designated periodic motion pattern is identified based on the motion sensing information, and
based on touch sensing information obtained by a touch sensor (216) of the wearable electronic device, a sensing axis of the motion sensor when the start of tooth-brushing is identified.

12. The method of any one of claim 8 to claim 11, further comprising identifying an end of tooth-brushing when the motion sensing information and/or the audio signal are not obtained for a designated time period.

13. The method of any one of claim 8 to claim 12, further comprising displaying, based on the identification of the end of tooth-brushing, information related to tooth-brushing from the start of tooth-brushing to the end of tooth-brushing on a display (260) of the wearable electronic device.

14. The method of any one of claim 8 to claim 13, further comprising transmitting the information related to tooth-brushing to an external electronic device via a communication module (219) of the wearable electronic device.

15. A non-transitory storage medium storing instructions, wherein the instructions are configured to enable, when executed by at least one processor (220) of a wearable electronic device (201), the wearable electronic device to perform at least one operation comprising:
obtaining motion sensing information via a motion sensor (212) of the wearable electronic device;
obtaining an audio signal corresponding to the motion sensing information via an audio sensor (218) of the wearable electronic device;
identifying a tooth-brushing hand motion type corresponding to the motion sensing information;
identifying an audio signal pattern corresponding to the tooth-brushing hand motion type; and
identifying a tooth-brushing hand motion corresponding to the motion sensing information and the audio signal, based on the tooth-brushing hand motion type and the audio signal pattern.
